Europäisches Patentamt

European Patent Office

Office européen des brevets

(11) Publication number: **0 042 064**
**B1**

# EUROPEAN PATENT SPECIFICATION

(45) Date of publication of patent specification: **16.09.87**

(21) Application number: **81103702.7**

(22) Date of filing: **14.05.81**

(51) Int. Cl.⁴: **A 61 K 39/00,** A 61 K 49/00, A 61 K 35/14

(54) Modulators of the immune system.

(30) Priority: **14.05.80 US 149737**
**06.05.81 US 256886**

(43) Date of publication of application:
**23.12.81 Bulletin 81/51**

(45) Publication of the grant of the patent:
**16.09.87 Bulletin 87/38**

(84) Designated Contracting States:
**AT BE CH DE FR GB IT LI LU NL SE**

(56) References cited:
**WO-A-80/00790**
**US-A-3 991 182**
**US-A-4 132 776**
**US-A-4 180 627**

**Journal of Immunology, vol. 124 (2), pages 885-892, 1980**

**CHEMICAL ABSTRACTS, vol. 85, no. 9, 30th August 1976 abstract no. 92022q, page 453 COLUMBUS, OHIO (US)**

**CHEMICAL ABSTRACTS, vol. 91, no. 11, 10th September 1979 abstract no. 89355u, page 593 COLUMBUS, OHIO (US)**

(73) Proprietor: **Gottlieb, Arthur A.**
**5915 Pitt Street**
**New Orleans Louisiana 70115 (US)**

(72) Inventor: **Gottlieb, Arthur A.**
**5915 Pitt Street**
**New Orleans Louisiana 70115 (US)**

(74) Representative: **Popp, Eugen, Dr. et al**
**MEISSNER, BOLTE & PARTNER**
**Widenmayerstrasse 48 Postfach 86 06 24**
**D-8000 München 86 (DE)**

(56) References cited:
**CHEMICAL ABSTRACTS, vol. 81, no. 19, 11th November 1974 abstract no. 118427v, page 367 COLUMBUS, OHIO (US)**

**CHEMICAL ABSTRACTS, vol. 88, no. 15, 10th April 1978 abstract no. 103120k, page 389 COLUMBUS, OHIO (US)**

**CHEMICAL ABSTRACTS, vol. 88, no. 25, 19th June 1978 abstract no. 187896d, page 3 COLUMBUS, OHIO (US)**

**CHEMICAL ABSTRACTS, vol. 91, no. 15, 8th October 1979 abstract no. 122024k, page 467 COLUMBUS, OHIO (US)**

EP 0 042 064 B1

(58) References cited:
CHEMICAL ABSTRACTS, vol. 91, no. 5, 30th July 1979 abstract no. 37394m, page 471 COLUMBUS, OHIO (US)

CHEMICAL ABSTRACTS, vol. 94, no. 19, 11th May 1981 abstract no. 154692e, page 473 COLUMBUS, OHIO (US)

CHEMICAL ABSTRACTS, vol. 93, no. 23, 8th December 1980 abstract no. 219228j, page 409 COLUMBUS, OHIO (US)

CHEMICAL ABSTRACTS, vol. 93, no. 25, 22nd December 1980 abstract no. 23662a, page 625 COLUMBUS, OHIO (US)

CHEMICAL ABSTRACTS, vol. 86, no. 21, 23rd May 1977 abstract no. 153824x, page 326 COLUMBUS, OHIO (US)

JOURNAL OF CLINICAL HEMATOLOGY AND ONCOLOGY, vol. 8, no. 4, 1978, G. PADDOCK et al.: "Purification and structural analyses of the transfer factor (TF)-like activity detected in vitro by leukocyte migration inhibition LMI", page 122

THE JOURNAL OF IMMUNOLOGY, vol. 122, no. 3, March 1979, (US), D.R. BURGER et al. "Human transfer factor: structural properties suggested by HPRP chromatography and enzymatic sensitivities", pages 1091-1098

CHEMICAL ABSTRACTS, vol. 92, no. 23, 9th June 1980, abstract no. 196150r, page 499, COLUMBUS OHIO (US)

SCANDINAVIAN JOURNAL OF IMMUNOLOGY, vol. 2, no. 4, 1973, K. KROHN et al.: "Fractions of human leukocyte dialysates and their transfer factor activity", page 450

FEDERATION PROCEEDINGS, vol. 35, no. 3, 1976, A.A. GOTTLIEB et al.: "Biochemical analysis of 2transfer factor", page 337

FEDERATION PROCEEDINGS, vol. 35, no. 3, 1976, L.A. ANDRON et al.: "Transfer factor in vitro: chromatography of components that enhance entigen induced lymphocyte transformation", page 337

JOURNAL OF CLINICAL INVESTIGATION, vol. 53, no. 6, 1974, J. NEIDHART et al.: "Preparative chromatographic technique for the active component of transfer factor", pages 55a and 56a

BIOLOGICAL ABSTRACTS, vol. 61, no. 10, 1976, abstract no. 55347, page 5738 PHILA. PA. (US)

BIOLOGICAL ABSTRACTS, vol. 61, no. 3, 1976, abstract no. 14396, page 1494, PHILA. PA. (US)

BIOLOGICAL ABSTRACTS, vol. 64, 1st November 1977, abstract no. 50550, page 4957, PHILA. PA. (US)

BIOLOGICAL ABSTRACTS, vol. 67, no. 3, 1979, abstract no. 15703, page 1542, PHILA. PA. (US)

(58) References cited:
CHEMICAL ABSTRACTS, vol. 80, no. 21, 27th May 1974, abstract no. 119055p, page 293, COLUMBUS OHIO (US)

CHEMICAL ABSTRACTS, vol. 85, no. 5, 2nd August 1976, abstract no. 31473r, page 279, COLUMBUS OHIO (US)

CHEMICAL ABSTRACTS, vol. 87, no. 9, 29th August 1977, abstract no. 66487s, page 392, COLUMBUS OHIO (US)

CHEMICAL ABSTRACTS, vol. 87, no. 13, 26th September 1977, abstract no. 100421c, page 468, COLUMBUS OHIO (US)

CHEMICAL ABSTRACTS, vol. 91, no. 7, 13th August 1979, abstract no. 54336m, page 492, COLUMBUS OHIO (US)

CHEMICAL ABSTRACTS, vol. 87, no. 23, 5th December 1977, abstract no. 182419v, page 448, COLUMBUS OHIO (US)

# 0 042 064

- **Description**

**Background of the invention**

The human immune system is highly complex and at present imperfectly understood. It is presently considered to have two principal aspects, humoral immunity, which is mediated by circulating antibodies, and cell-mediated immunity, which, as the name implies, is mediated by lymphoid cells. Humoral immunity is transferred from an immune donor to a non-immune recipient by means of serum immunoglobulins, and such serum-mediated transfers result in immune responses, which are manifest almost immediately. Cell-mediated immunity is transferred by means of peripheral blood leukocytes. Immune responses transferred by cells develop slowly, over a period of several hours.

A typical manifestation of cell-mediated immunity is the delayed hypersensitivity reaction, abbreviated "DH" herein. A DH skin reaction is observed when the appropriate antigen is injected subcutaneously. Within 24 to 48 hours, local inflammation (erythema) and a swelling and thickening (induration) are observed in a sensitive individual, and the degree of sensitivity may be measured by the size and severity of the reaction. The DH reaction also presents characteristic histological findings, specifically, perivascular infiltration of lymphocytes and monocytes in the inflamed area. The cells seen at the site of a DH reaction are derived from the peripheral blood leukocyte population.

The mechanisms of cell-mediated immunity are as yet incompletely understood. It is known that the cells which mediate the response are capable of responding in a variety of ways to an antigenic challenge. These responses include: proliferation of cells bearing specific sensitivity to a given antigen; the induction and multiplication of cells mediating a variety of immune functions, including antibody production; and reactions against foreign cells and tumors. The quality and quantity of these response patterns is affected by many factors, including hormones of the thymus and adrenal cortex; interferons; and other effectors of cellular responses, including histamine, serotonin, and the prostaglandins.

The present invention relates to the discovery of modulators of the immunity system, isolated from dialyzed extracts of leukocytes; profoundly affecting the quality and quantity of cell-mediated immunity responses; and useful in the treatment of a variety of clinical conditions characterized by overreaction or by inadequate reaction to a specific antigen, and in the alleviation of certain anergic conditions.

**Prior art**

In 1954, H. S. Lawrence reported that a lysate prepared from the leukocytes of tuberculin-sensitive donors could transfer that sensitivity to tuberculin-nonreactive recipients (Journal of Clinical Investigation, 33, 951 (1954)). See Lawrence, H. S., The Harvey Lectures, 68, 239 (1974) for a later review of that work, and Transfer Factor; Basic Properties and Clinical Applications, M. S. Ascher, A. A. Gottlieb (the applicant) and C. H. Kirkpatrick Eds., Academic Press, Inc., New York (1976) and Immune Regulators in Transfer Factor, A. Kahn et al, Eds., Academic Press, Inc., New York (1979) for collections of state-of-the-art papers. The transfer of sensitivity was presumed to be due to a factor in the leukocyte lysate which was later given the operational term "transfer factor" (Lawrence, H. S., commenting on paper of Najarian, J. S., and Feldman, J. D., in Cell-Bound Antibodies (B. Amos and H. Koprowski, Eds.). The "transfer factor" phenomenon is demonstrable only in human beings. Consequently, research progress in isolating and characterizing the active principle has been slow.

To demonstrate transfer factor, two patients must be identified, a donor, known to display a DH reaction to a given antigen, and a recipient, known to give no DH reaction when challenged with the same antigen (and therefore presumably lacking cell-mediated immunity to that antigen). Leukocytes prepared from the blood of the donor are disrupted, and the cell contents dialyzed. The concentrated dialysate, in a suitable buffer, is injected subcutaneously in the forearm or other convenient location of the recipient. After about two days, the recipient is then challenged by a subcutaneous injection of the antigen at the same or another location. A typical DH reaction can be observed about one week later. Immunity thus transferred reportedly may persist in the recipient for as long as two years.

Progress in fractionating and characterizing transfer factor has been impeded by the lack of associated structural or chemical criteria and by the fact that the phenomena observed after fractionation are often qualitatively different from the phenomena induced by the original dialysate. Although the term "transfer factor" appears in literature as applied to fractionated preparations, and as monitored by criteria other than a DH skin response, it is unclear whether such terminology indeed refers to a single biochemical entity or to an activity that represents a single biological function. As used herein, the term "transfer factor" is restricted to the crude leukocyte dialysate as isolated by Lawrence and having an activity as originally characterized by Lawrence, i.e., the ability to transfer immunity to a specific antigen from a highly sensitive donor to a non-sensitive recipient.

Prior art fractionation of human transfer factor has demonstrated the presence of a variety of active materials. Vandenbark, A. A. et al, J. Immunol., 118, 636 (1977), reported the results of Sephadex G-25 (trademark Pharmacia, Inc., Uppsala, Sweden) chromatography of the dialysate of leukocyte extracts. Biological activity in vivo was found only in association with two major peaks of optical density at 254 nm or 280 nm. One such peak fraction was reported to transfer significant skin test reactivity from antigen responsive donors to previously non-reactive recipients. The other peak produced spontaneous activity (a dermal response without added antigen) and also significantly increased dermal response when combined

3

with antigen. The peak fractions were further fractionated by isoelectric focusing and by reverse phase chromatography using a solvent system of one percent acetic acid or five percent methanol on a column bed packed with octadecyl silane resin. However, results of biological activity tests following sub-fractionation were not reported.

Gottlieb, A. A., et al, in Transfer Factor: Basic Properties and Clinical Applications, at page 263, introduced the use of fluorescamine as a means for monitoring the fractionation of leukocyte dialysates. Fluorescamine (also termed "Fluram", trademark Hoffman-LaRoche, Inc., Nutley, N. J.), Bohlen, P. et al, Arch. Biochem. Biophys., 155, 213 (1973) reacts with substances containing primary amino groups to yield highly fluorescent products, providing a highly sensitive assay for proteins and other compounds bearing primary amino groups. The dialyzable leukocyte extract material was fractionated on a Sephadex G-10 (trademark Pharmacia, Inc., Uppsala, Sweden) column and biological activity was found associated with a major fluorescamine-reactive peak. Delayed hypersensitivity responses were observed whether or not antigen was added, although response was somewhat greater in the presence of antigen.

Dialysable leukocyte extracts were further fractionated by Gottlieb, A. A., et al., as reported in J. Reticuloendothelial Soc., 21, 403 (1977). The extracts were first subjected to differential molecular weight dialysis, using dialysis membranes having nominal molecular weight cutoffs of about 12,000 and about 3,500, respectively. Material passing through the latter membrane, having a molecular weight range generally less than 3,500, was termed the "S" fraction, while material in the general molecular weight range of 3,500 to 12,000 was designated the "L" fraction. Both fractions were subjected to gel filtration on Sephadex G-10. Biological activity was again associated with the fluorescamine reactive peak functions. However, activity did not appear to be a function of the immunologic state of the donor. In addition, two distinguishable effects were observed. The first was an induction of DH response in the absence of added antigen and independent of the immunologic state of the donor or recipient. Such activity is referred to herein as "inducer", as defined infra. The second activity was the ability to augment intradermal reactions to antigens to which the recipient was sensitive but not to antigens to which the recipient had no prior exposure. This activity, found in the fluorescamine reactive chromatographic fractions, appeared to augment the recipient's DH response to an antigen to which he was already sensitive. In both instances, the sensitivity of the donor appeared irrelevant. Additional fractionation of the S fraction on hydroxylapatite chromatography showed that the biological activity producing a DH response in the absence of added antigen was not associated with the main polypeptide fraction, as measured by reaction with fluorescamine.

Additional data presented by Gottlieb, A. A., et al, in J. Immunol., 124, 885 (1980), were presented on the fractionation of "inducer" (producing a DH reaction in the absence of added antigen) and the "amplifier" (producing a DH reaction in the presence of antigens to which the recipient is known to be sensitive), by means of a long, 150 cm, Sephadex G-10 column. "Amplifier" remained associated with the main fluorescamine reactive fractions, while "inducer" was not. Upon further fractionation on hydroxylapatite, the "amplifier" activity remained associated with the fluoroescamine-reactive peak. Controlled studies demonstrated that the reported activities were not found in similarly treated lysates of red blood cells nor was any activity associated with a saline solution processed according to the same purification procedure.

Gottlieb, A. A., et al. (Immune Regulators in Transfer Factor), A. Kahn, C. Kirkpatrick and Hill. Eds., Academic Press, Inc., New York (1979), page 339, in a report unaccompanied by data, suggested that an additional activity, termed "suppressor", could be separated from an "augmentor" activity by hydroxylapatite chromatography. The "augmentor" was found in the "S" dialysis fraction and was always associated with the major fluorescamine-reactive peak. The "augmentor" fraction was thought to be systemically effective in previously BCG-exposed anergic patients. In addition, a "suppressor" activity found in the "L" dialysis fraction was stated to elute at a higher salt concentration than the fluorescamine-reactive material, on hydroxylapatite chromatography. In another study where dialyzed leukocyte extracts were fractionated, Wilson, G. B. et al, Ja. Lab. Clin. Med., 93, 819 (1979) reported four activities affecting in vitro leukocyte migration: two antigen-independent activities (producing a response in the absence of any antigen), an antigen-dependent specific inhibitor and an antigen-dependent enhancer. The antigen-dependent inhibitor had properties in common with "transfer factor" since only antigen of the appropriate donor specificity caused significant inhibition of in vitro leukocyte migration. The antigen-dependent enhancement of leukocyte migration was not characterized with respect to antigen specificity. That activity, however, is not known to have any relationship to any in vivo function of the human immune system nor any predictable therapeutic utility.

Summary of the invention

This invention relates to modulators of the human immune system derived from human leucocytes, purified substantially free of fluorescamine-reactive substances, having the activity of increasing or decreasing a delayed hypersensitivity skin response to antigens in a human being. The invention further relates to a composition for increasing responsiveness of the human immune system to said antigens and to a method of purifying from an extract of human leucocytes L-suppressors or amplifiers 1 to 6 and S-suppressor.

A modulator as herein defined is any substance that effects direct or indirect response of an animal or human body, portion thereof or matter taken therefrom, to antigens to which said body has been previously exposed, where such response is specifically attributable to the function of the immunity system of said animal or human. Substances having general effects which may include effects on the immune response are therefore not subsumed within the term "modulator", as herein defined. These modulators described herein manifest their activity in a DH skin reaction test, and therefore appear to exert their primary effect on the cell-mediated immunity system. It will be understood, however, that the described modulators have broad effects on the entire immunity system, and may affect the humoral immunity system. For purposes of discussion herein, the terms "transfer factor", "inducer", "amplifier", and "suppressor" are each defined in terms of their respective activities eliciting a DH response in a skin reaction.

The term "transfer factor" denotes a dialysate of a crude leukocyte extract, a described supra. A "transfer factor activity" is manifest when the transfer factor preparation is made from leukocytes of a donor known to be sensitive to a given antigen and is injected subcutaneously in the skin of a recipient known to be insensitive to the same antigen. The recipient is challenged, at a later time, with the antigen, and a DH response is observed. Normally, the recipient, in the absence of injected transfer factor activity, would be unresponsive. It will be understood that transfer factors are not modulators, as defined supra, since the effect of a transfer factor is observed in a recipient which has not previously been exposed to a given antigen. Furthermore, transfer factor effects are specific with respect to a given antigen, whereas the amplifiers and suppressors herein described exert non-specific effects with respect to any antigen to which the recipient was previously exposed.

"Inducer" is defined as that activity producing a DH response in the absence of added antigen and irrespective of the sensitivities of donor and recipient.

"Amplifier" is characterized by the production of a greater-than-normal response in a sensitive recipient, following injection of the antigen to which the recipient is sensitive. Amplifier activity is not dependent upon the specific immunological sensitivity of the donor.

"Suppressor" activity is observed in a sensitive recipient when the suppressor and an antigen to which the recipient is sensitive are injected together, with the result that the recipient manifests a less-than-normal DH response.

In the present invention, modulators of the human immune system have been isolated from dialysates of leukocyte extracts. Six such modulators have amplifier activity and two have suppressor activity. Amplifiers are considered useful in the treatment of energic conditions and conditions of immune hyposensitivity, both local and systemic, while suppressors are considered useful for the preparation and treatment of local hypersensitivity conditions such as poison ivy. These amplifiers are designated amplifiers 1—6. The suppressors are identified as the S-suppressor and the L-suppressor.

Detailed description of the preferred embodiment

A principal contribution of the present invention lies in the discovery of substances whose function is to modulate immune responsiveness. Present techniques have permitted identification of modulators which amplify or suppress the response to antigens to which an individual human being has been previously exposed. However, it is evident that a variety of modulating functions may be manifested by such substances, including duration of response, threshold of sensitivity, and type of response, whether inflammatory, proliferation of lymphocytes or production of circulating antibody. Clearly such modulating functions will be defined in terms of the test system used to detect and measure them.

The modulators herein described are therefore but part of a system of modulator substances which constitute a natural means of intracellular communication between components of the immune system, whereby the state of immune responsiveness is continually monitored and modified in accordance with the level and activity of current antigen challenge.

Amplifier 1 is characterized as having an accelerating and augmenting effect on the DH skin response of recipients sensitive to a given antigen. The reactions produced by amplifier 1 administered with antigen reach peak intensity at about 6 to 14 hours after subcutaneous injection and fade rapidly thereafter. (In contrast, normal DH response, in the absence of amplifier, reaches a peak 24 to 30 hours after injection of antigen). Amplifier 1 is separable from the other modulators of the present invention by reverse phase chromatography using an octadecyl silane column eluted with a 0 to 100% (v/v) ethanol-in-water gradient. Amplifier 1 is elutable from the column by at least 15% (v/v) ethanol in water and the majority of this amplifier is elutable from about 15% (v/v) to about 20% (v/v) ethanol in water. Amplifier 1 is not demonstrably reactive with fluorescamine, and is separated from the main peak of fluorescamine reactivity on reverse-phase chromatography. Amplifier 1 passes through a dialysis membrane having a nominal molecular weight (M. W.) cutoff of 3,500.

Amplifier 2 also causes both an accelerated and an augmented response to antigen, although the degree of acceleration is somewhat less rapid than the response to amplifier 1. Reaction sites are more circumscribed than those produced by amplifier 1 plus antigen, and they persist considerably longer (up to seven days). Most surprisingly, maximal amplifying activity is observable only at an optimum concentration, with greater-than-optimal concentrations giving reduced amplification or even suppression of the DH response. Amplifier 2 is elutable from octadecyl silane by at least 45% (v/v) ethanol-water solution, and the majority of this amplifier is elutable from about 45% (v/v) to about 53% (v/v) ethanol in

5

water. It is not demonstrably reactive with fluorescamine, and is separable from the other described modulators isolated from a dialysate of leukocyte extracts and also from the major peak of fluorescamine-reactive material in such extracts by reverse phase chromatography. The molecular weight and size of amplifier 2 are such that it passes through a dialysis membrane having a nominal molecular weight cutoff of about 3,500.

Amplifiers 3, 4 and 5 cause augmented responses to antigen. Dilution studies indicate that the activity of amplifiers 3 and 5 is enhanced by dilution, in that the activity of amplifier 4 may also be enhanced, although the data are somewhat more equivocal. Amplifiers 3, 4 and 5 are elutable from octadecyl silane in at least 70% (v/v), 80% (v/v) and 89% (v/v) ethanol-water solutions, respectively. The majority of these amplifiers are elutable in the following concentration ranges of ethanol in water: Amplifier 3, 70% (v/v) to 74% (v/v); Amplifier 4, 80% (v/v) to 84% (v/v); Amplifier 5, 89% (v/v) to 94% (v/v). None of these amplifiers is demonstrably reactive with fluorescamine and all are separable from each other and from the other modulators described herein by reverse phase chromatography. Amplifiers 3, 4 and 5 are each of such size as to pass through a dialysis membrane having a nominal molecular weight cutoff of about 3,500.

Amplifier 6 causes augmented response to antigen in a DH reaction and has also been shown to have systemic effects. In common with most of the amplifying modulators described herein, except amplifier 1, there is a dose optimum for maximal response, which is observed by comparing the effects of increasing dilution of the sample as isolated. Amplifier 6 is elutable from octedecyl silane in 100% (v/v) ethanol. It is not demonstrably reactive with fluorescamine and is separable from the other modulators described herein by reverse-phase chromatography. The molecular weight and size of amplifier 6 are such that it passes through a dialysis membrane having a nominal molecular weight cutoff of about 3,500.

The S-suppressor is isolated from the dialysate passing through a membrane having a nominal 3,500 M.W. cutoff. S-suppressor is identifiable and at least partly separable from amplifiers 1 and 2 by reverse-phase chromatography and elution with a 0 to 100% (v/v) ethanol-in-water gradient. S-suppressor is elutable from octadecyl silane by ethanol in water ranging from 67% to 100% (v/v), and is clearly present in fractions eluting from 96% (v/v) to 99% (v/v). S-suppressor is not demonstrably reactive with fluorescamine, as judged by its separability from the major fluorescamine-reactive peak on reverse-phase chromatography. Suppression of a DH skin reaction is manifested when S-suppressor is injected before or concurrently with a test antigen to which the recipient gives a DH response. Suppression is reversible or shortacting, in effect delaying the onset of DH about 72 hours. Suppression is not observed when amplifier 2 is injected concurrently with S-suppressor.

The L-suppressor is found in that fraction of the leukocyte extract passing through dialysis membrane having a nominal 12,000 M.W. cutoff, but retained by membrane having a 3,500 M.W. cutoff. Similar in activity to the S-suppressor, the L-suppressor activity is reversible, having a suppressing effect lasting about 72 hours. L-suppressor is not fluorescamine-reactive, as judged by the fact that it is separable from the major fluorescamine reactive peak upon hydroxylapatite chromatography.

In the following examples, procedures are described wherein materials were obtained from human donors and test measurements were made on human recipients. The procedures and reagents used herein were chosen to provide sterile and non-toxic products for human treatment. The sensitivities of donors and recipients to selected antigens were tested in advance.

Example 1

Purification procedure

A. Preparation of Leukocytes. Leukocytes were prepared by standard methods, employing either fractionation of whole blood samples or leukophoresis. In the former method, a 450 ml sample of whole blood was fractionated by sedimentation at 1×g in Macrodex (trademark Pharmacia Corporation, Piscataway, N. J. for 6%

$$\left( \frac{W}{V} \right)$$

Dextran 70 in normal saline) to separate red blood cells from leukocytes. Approximately $1-2 \times 10^9$ leukocytes were recovered by this method. Leukophoresis was preferred for obtaining larger amounts of cells. A Haemonetics (trademark Haemonetics Corp., Braintree, Mass.) Model 30S cell separator was employed. The system was primed with 30 cc of 46.7% Trisodium Citrate (Haemonetics, Braintree, Mass.) and 500 ml of 6%

$$\left( \frac{W}{V} \right)$$

Volex (trademark McGaw Co., Irvine, Calif.) a high-molecular-weight starch preparation used to enhance recovery of leukocytes from the peripheral blood and removal of red blood cells from the final leukocyte preparation by sedimentation. In either case, leukocyte rich plasma was obtained following a 60-minute incubation at 37°C. The leukocytes were recovered from the plasma by centrifugation at 400×g for 15 minutes followed by three washes with 0.5 M saline. After washing, the leukocyte pellet was stored frozen at −20°C. An average yield of leukocytes from six passes in the cell separator was $1 \times 10^{10}$ cells.

B. Dialysis. Leukocyte extracts were prepared under sterile conditions. Leukocyte pellets were resuspended in 10 ml of 5 mM ammonium bicarbonate, then subjected to 10 cycles of freeze-thawing in a dry ice and acetone bath. The lysate was first dialyzed against 5 mM ammonium bicarbonate, using cellulose dialysis tubing having a nominal 12,000 M.W. cutoff (Arthur H. Thomas, Inc., Chicago, Ill.). After three changes of buffer, the combined dialysates were lyophilized, redissolved in a small volume of ammonium bicarbonate, and dialyzed in cellulose tubing of nominal 3,500 M.W. cutoff, against 5 mM ammonium bicarbonate as before. The retentate from the second dialysis, termed the "L" fraction herein, and the dialysate, termed the "S" fraction herein, were each lyophilized and stored frozen at −20°C until further use.

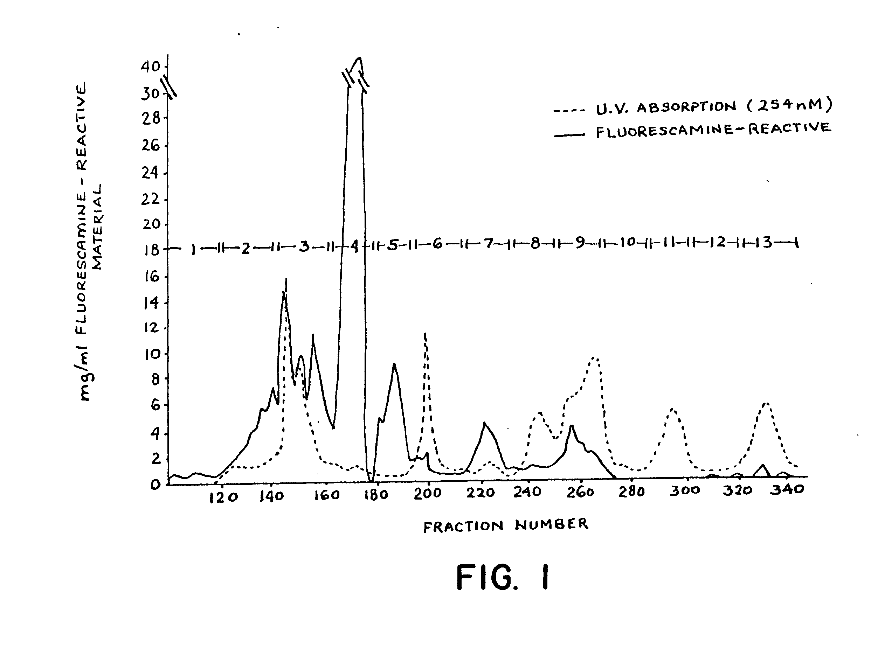

C. Gel Filtration. Sephadex G-10 (trademark Pharmacia, Inc., Uppsala, Sweden) was swollen overnight in 5 mM ammonium bicarbonate, then autoclaved. After removal of the fines, a 1.5 cm×151 cm column was prepared and equilibrated in 5 mM ammonium bicarbonate. The column was loaded with 400 mg of fluorescamine-reactive material of the redissolved S fraction (based upon bovine serum albumin as the assay standard, according to Bohlen, P. et al, Arch. Biochem. Biophys., 155, 213 (1973). The sample was eluted with 5 mM ammonium bicarbonate at a flow rate of 13 ml/hr. One total column volume (254 ml) was collected in 0.8 ml fractions. Ultraviolet absorption at 254 nm and fluorescamine reactivity, based upon 100 µl aliquots, were measured. The results are shown in Figure 1.

A major fluorescamine-reactive peak was observed. The pattern was essentially constant from one individual donor to another. The location of modulator activities in relation to the fluorescamine reactivity was discovered by assays of DH response over a series of 10-fold dilutions. The nature of the DH response varied in an unexpected manner with dilution. Undiluted samples in the region of the fluorescamine peak gave a reduced DH response to an antigen relative to the control response to antigen alone. Surprisingly, at dilutions of $10^{-2}$ and $10^{-3}$, amplification of the DH response, relative to the control, was observed. Even more surprisingly, at dilutions of the order of $10^{-5}$, suppression of the DH response was observed. The fractions displaying such unusual response properties, termed "modulator-assayed" fractions herein, were selected and subjected to additional purification. From such experiments, the activities of interest ("modulator-assayed") were located in a region containing the major fluorescamine-reactive peak and a smaller fluorescamine-reactive peak preceding the major fluorescamine-reactive peak. For subsequent purification steps, fractions 152 through 178 (shown in Figure 1) were pooled and lyophilized. The possibility exists that final yields can be improved by pooling additional fractions on either side of those chosen. The optimal choice of fractions is not as yet determined.

In some experiments, gel filtration was carried out using a shorter (80 cm) column of Sephadex G-10. The results were comparable, except that the longer column gave improved resolution. In particular, an antigen independent inducer was separated from the main fluorescamine-reactive peak on the longer column.

As an alternative to gel filtration, purifications could be carried out by high-performance gel exclusion chromatography, using a 1×25 cm column of sulfonated polystyrene divinyl benzene having a 5,000 molecular weight exclusion limit (Shodex S-802/S, manufactured by Showa Denko KK, Tokyo, Japan), eluted with water at the rate of 0.8 ml/min.

D. Reverse Phase Chromatography. Further purification was carried out by reverse-phase high pressure liquid chromatography using an octadecyl silane column eluted with a 0 to 100% (v/v) gradient of ethanol in water. The column was loaded with 30 mg of sample (based upon fluorescamine reactivity) and eluted at the rate of 0.5 ml/min, collecting 1 ml fractions. In some preparations, the ethanol-water gradient was followed by elution with 100% ethanol at the same rate.

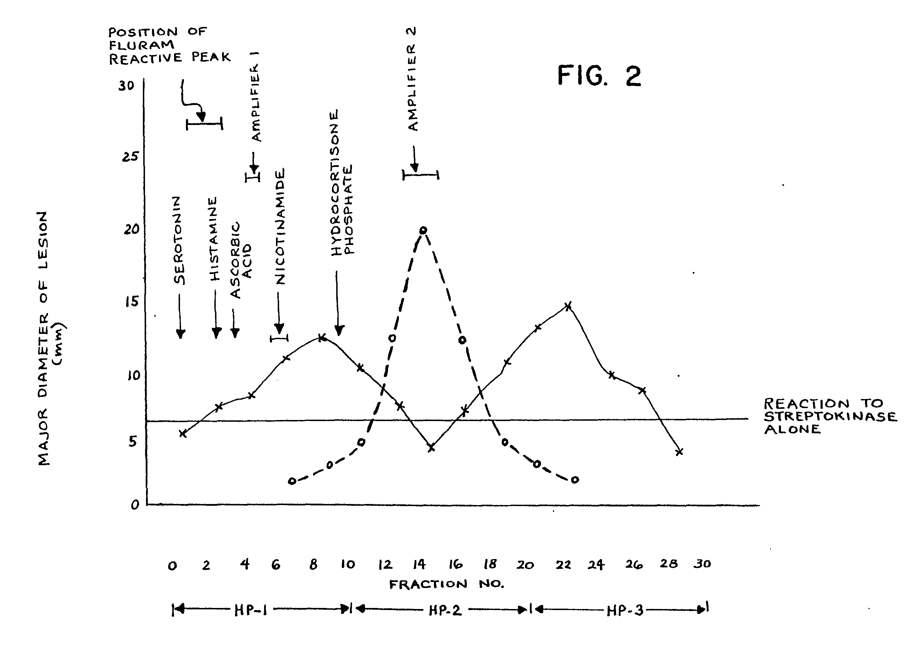

The fractions were lyophilized and redissolved in 0.5 ml normal saline. The fractions, 0.1 ml each, were injected intradermally in combination with an antigen to which the recipient had been previously shown to be sensitive, and the dermal responses at 24 hours were measured. The results are shown in Figure 2.

In the separation shown in Figure 2, the material was obtained from a donor who was insensitive to the antigen streptokinase (hereinafter "SK"). The recipient was known to be sensitive to streptokinase. The extent of his dermal reaction to SK in the absence of any modulators is presented as a horizontal line in Figure 2. DH responses above the line show amplifier activity, while responses below the normal level suggest suppressor activity. For reference, the positions are indicated in Figure 2 of the fractions containing fluorescamine-reactive material and of various small molecules (serotonin, histamine, ascorbic acid, nicotinamide and hydrocortisone phosphate) known to be vasoactive, inflammatory, or anti-inflammatory. The solid line shows the dermal reactivity of individual fractions in the concentrations obtained from the gradient.

The results of individual fractions were compared with the activity tests made with pooled fractions (0—10, 10—20, and 20—30) administered at several dilutions. Paradoxically, at higher dilution, the pooled 10—20 fractions manifested the maximum observed amplifier activity. The dilution effect of individual fraction 5, having maximal amplifier 1 activity, and fraction 14, were measured in an individual sensitive to SK. The results were expressed by measurement in millimeters of the dimensions of the regions of erythema and of induration, at 7 hours, 12 hours and 24 hours after injection of antigen and modulator. The modulators were diluted in normal saline. These data are given in Table 1.

In the case of amplifier 1 (fraction 5 of Figure 2), strong acceleration and amplification effects were observed at all tested levels of dilution, suggesting saturation of the test site at all concentrations studied. However, with fraction 14, significant amplification was observed only upon dilution by a factor of 10 to 1,000. Therefore, a clear-cut optimum concentration for amplifier 2 activity was observed. A degree of acceleration was also observed, although less pronounced than that produced by amplifier 1.

The two apparent peaks of amplifier activity obtained by the measurement of undiluted samples in Figure 2 do not necessarily reflect the true positions of the amplifiers in all preparations. There are several reasons for this. First, different donors have different levels of the modulators in their leukocytes. They may also have other substances in their leukocytes that affect the preparation. There is also biological variability in the test recipient. Finally, there is the paradoxical effect of concentration in the case of amplifier 2. In particular, in Figure 2, the decreased activity in the region of fractions 12 through 18 is clearly a consequence of above-optimal concentrations of amplifier 2 in this region. When optimally diluted, a peak of amplifier 2 activity should be observable in the region of fractions 14 through 16. This conclusion is represented by the dashed line in Figure 2, which is a hypothetical curve of amplifier 2 activity measured at optimum dilution.

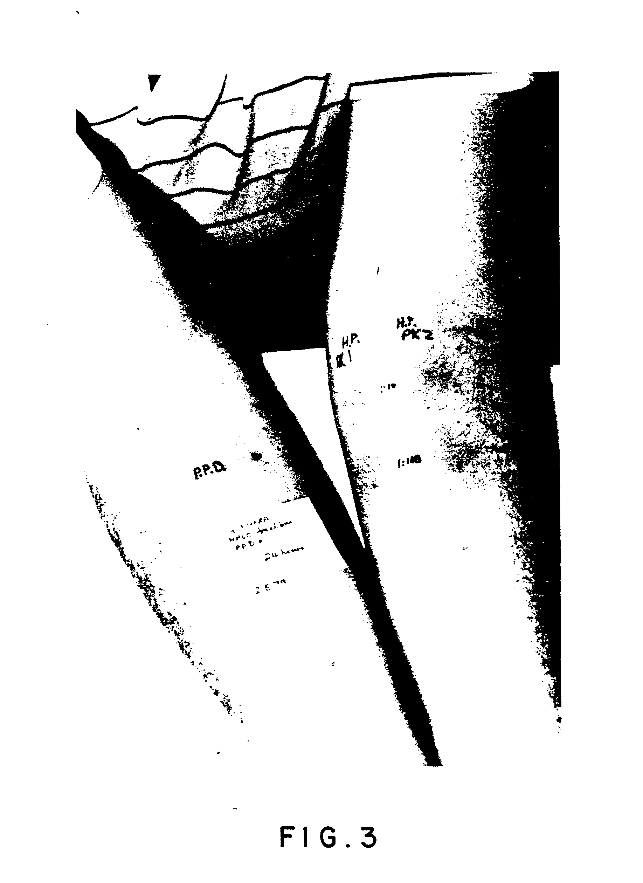

Pooled fractions 21—30 had S-suppressor activity. When injected concurrently with antigen the S-suppressor effectively prevented the appearance of a DH response at 6 or 24 hours after injection. In contrast, a clear-cut DH response was observed at 24 hours where antigen was injected alone, and an even stronger response was observed where amplifiers 1 or 2 were also present. The results are shown in Figure 3. The patient's left arm received a series of subcutaneous injections of PPD mixed with pooled fractions of the gradient shown in Figure 2 at 1:10 and 1:100 dilutions, as indicated on the patient's arm: HP-1 (pooled fractions 1—10) designated HP-PK-1 on the patient's arm; HP-2 (fractions 11—20) designated HP-PK-2; and HP-3 (fractions 21—30) designated HP-PK-3. The control injection of PPD alone was made on the patient's right arm. Increased inflammation, compared to control, is observable at the sites injected with pooled HP-1 and HP-2, due to the presence of amplifiers in these fractions. However reduced inflammation is observed at HP-3 treated sites, due to the presence of suppressor activity, which appears to predominate over the activity of any amplifiers in this fraction. The S-suppressor activity was observed in pooled fractions 21—30 of the reverse phase chromatographic separation, in most donors. In some instances, S-suppressor activity could be found in single fractions, e.g., fraction 29 of Figure 2. However, suppressor activity of single fractions was not consistently observed in all preparations, and presence of such activity proved to depend upon the donor individual.

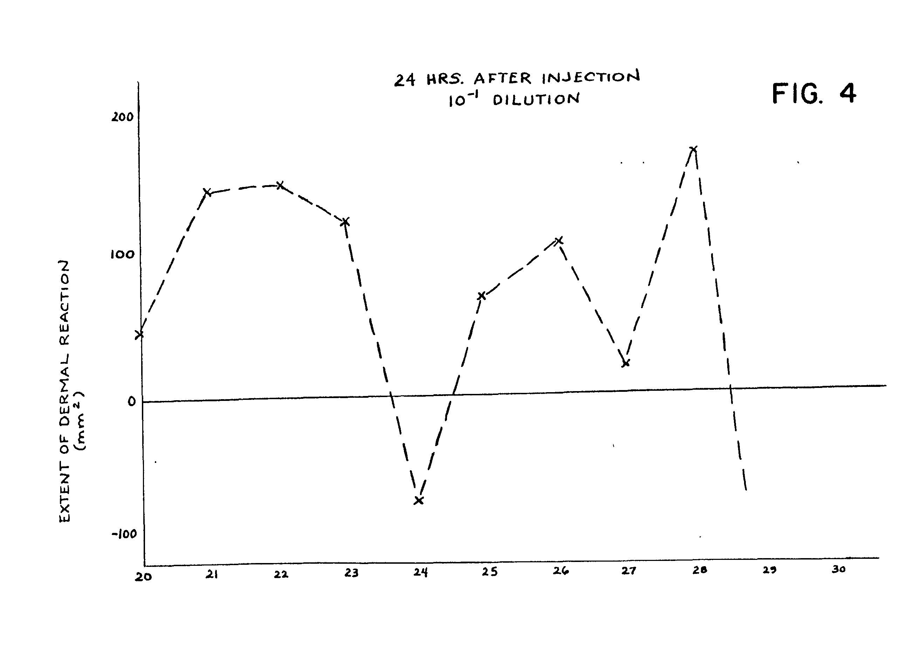

When individual fractions in the region from 20 to 30 of the reverse phase chromatographic separation were assayed at several dilutions, the existence of amplifiers 3, 4 and 5 became apparent. The peak of amplifier 3 activity appeared in fractions 21 and 22, while amplifier 4 activity was centered in fraction 25, and amplifier 5 activity was found chiefly in fractions 27 and 28, S-suppressor was found in fractions 29 and 30. The results are shown in Figure 4, showing analysis of a representative chromatographic run. In Figure 4, DH response relative to controls was plotted as the extent of dermal reaction, defined as $(a \times b) - (a^* \times b^*)$, where a and b are diameters in millimeters of the erythematous lesion resulting from injection of fraction plus antigen, measured along mutually perpendicular axis, and $a^*$ and $b^*$ are the respective diameters of the control lesion resulting from injection of antigen alone. The extent of dermal reaction is therefore the difference between the approximate areas of lesions produced by antigen alone and antigen mixed with modulator. Values appreciably below the control level of zero, such as those observed in fractions 29 and 30, indicate suppressor activity, while values appreciably above zero indicate amplifier activity. It should be noted that the pattern in Figure 4 is compatible with that of Figure 2, when one considers the results of the odd-numbered fractions which were the only fractions assayed in the experiment shown in Figure 2.

Amplifier 6 was eluted from the reverse-phase column after the ethanol-water gradient had reached a concentration of 99.9% ethanol, by continuing the elution of the column with 100% ethanol at 0.5 ml/min. collecting 1 ml fractions. Amplifier 6 was detected in tubes 32 through 34 under these conditions, with the greatest apparent concentration in tube 33.

Amplifier 6 caused an accelerated and augmented response to antigens to which the recipient was sensitive. A dose equivalent to the yield from $2 \times 10^8$ leukocytes in the described purification improved, and in some instances restored, the DH response of patients rendered weakly responsive by intercurrent illness, to antigens to which they were previously exposed. In general, the response to amplifier 6 were enhanced by dilution, although the optimal dilution was variable with the individual recipient and perhaps depended upon the antigen as well. Table 4 shows the results of two dilution experiments. In part A, a streptokinase-sensitive recipient received 2.5 units of streptokinase simultaneously with the amplifier. 1.0 ml fractions of amplifier collected from the reverse-phase column was lyophilized and redissolved in 0.3 ml normal saline. 0.1 ml of the indicated fractions, diluted to the extent shown, with normal saline, was injected. In part B, a PPD-sensitive recipient was injected with 0.05 ml of a 1/10 dilution of standard Aplisol PPD, together with the dilution of amplifier 6 indicated, in 0.1 ml saline, or with 0.1 ml of normal saline alone as a control.

Material with amplifier activity is extractable from aqueous solution with ether. The extractable activity requires dilution for maximal effect. The ether extract is therefore, presumed to contain at least some of the described amplifiers. However, it is not known which of the described amplifiers are ether-extractable. Reverse-phase chromatographic separation of the above-described modulators can be carried out using a variety of known reverse-phase column materials known in the art. While conditions of elution may vary, the optimal conditions for separating the above-described modulators will be readily determined by those of ordinary skill in the art.

E. Fractionation of the L Dialysis Fraction. The L dialysis fraction was further fractionated by chromatography on hydroxylapatite. After removal of fines, hydroxylapatite previously equilibrated with 5 mM ammonium bicarbonate was packed in a 1.5×20 cm column. The L fraction, lyophilized and redissolved in 0.05 M ammonium bicarbonate, was applied to the column. The column was eluted with a gradient of 0.05 M to 0.2 M aqueous ammonium bicarbonate (115 ml), followed by a gradient of 0.2 M to 0.6 M ammonium bicarbonate (65 ml). 1 ml fractions were monitored for absorbance at 260 nm and reactivities with fluorescamine. Individual fractions were pooled in seven combined fractions spanning most of the gradient. The combined fractions were analyzed for biological activity by injecting each fraction intradermally in the presence of an antigen to which the recipient individual was sensitive. The results are shown in Table 2. Relative DH response is indicated in Table 2 by the diameter of the region of induration, in millimeters, measured at 25 and 43 hours, compared to a control-reaction site wherein antigen alone was injected. Fractions 450 and 451, eluting between 0.1 M and 0.15 M ammonium bicarbonate, strongly suppressed the DH reaction for at least 43 hours. At 72 hours, reactions measuring 10 mm×10 mm were seen at sites receiving fractions 450 and 451, indicating that the suppressor activity is reversible over time.

Example 2
Characterization and control experiments

A. Dialyzed red blood cell extracts were prepared in the same fashion that leukocyte extracts were prepared in Example 1B. Differential dialysis, column chromatography on Sephadex G-10, were performed as described in Example 1. No immune modulator activity was observed in the resulting fractions. Therefore, the observed biological activity was not introduced by the extraction and purification steps. In addition, extracts of plate-lets subjected to identical purification steps, were devoid of modulator activity. Some of the purified amplifiers have been re-chromatographed and found to behave in essentially the manner observed during initial purification.

B. In view of the significant body of prior art dealing with transfer factor, it was important to show, as unequivocally as possible, that the observed amplifications of recipient sensitivity were not in fact due to the transfer of a low-level sensitivity, previously undetected in the donor, which, when concentrated, would appear as amplified sensitivity in a recipient. The experimental strategy used herein was based upon testing for antigens having geographically localized prevalence or having a medically traceable source. PPD, a purified protein derivative of tubercle bacillus, is both medically traceable and geographically localized. Sensitivity to PPD occurs in individuals with a prior history of vaccination with BCG (Bacille Calmette-Guerin), widely used to immunize against tuberculosis in Europe. However, its use has not been approved in the United States. Histoplasmin is a geographically-localized antigen. Sensitivity to histoplasmin is widespread in the southern United States and in tropical regions, where histoplasmosis is endemic, but is not found in northern Europe.

In the control experiment, the donor, a native of the south estern United States, was skin test sensitive to histoplasmin, but non-reactive to PPD. The recipients were lifetime residents of the United Kingdom with either a demonstrable skin test sensitivity to PPD or prior history of vaccination with BCG. The amplifier preparation employed in this experiment was purified as described, through the Sephadex G-10 fractionation step, except that a shorter, 80 cm column with somewhat lower resolution was employed. Consequently, test material was a mixture of amplifiers 1—6 and S-suppressor, in additional to fluorescamine-reactive material. The results are nevertheless significant as proof of the lack of transfer factor activity in the preparation.

The results are shown in Table 3. Two recipients were each tested with two fluorescamine-reactive peak fractions (number 33 and 39 of the Sephadex column).

Each fraction, 2.4 mg based upon fluorescamine reactivity, was injected either alone or with 25 units of PPD. Fourteen hours after the initial injection, the sites which had not previously received antigen were challenged with histoplasmin, 0.1 ml of Histoplasmin Antigen (preparation of Parke-Davis Corp., Detroit, Mich., sold as 1/100

$$\left(\frac{W}{V}\right)$$

dilution in normal saline). Control sites, with PPD or histoplasmin injected alone, at the appropriate time, were also prepared. In Table 3, the intensity of the dermal skin reaction is expressed in terms of the diameter in mm of the zone of induration surrounding the injection site. The data show that the recipients lacked any capacity to react with histoplasmin, either before or after injection of leukocyte extract fractions. Both the accelerating and augmenting aspects of amplifier activity are observable. On the other hand, no transfer of histoplasmin sensitivity is observable.

C. An important aspect of the amplifier activities described herein is that all of these tested, including the accelerating (amplifier 1) and augmenting (amplifier 2) activities and amplifier 6 are systemically effective. Furthermore, the systemic effects can be observed in anergic patients (those who have lost their normal immune responsiveness due to illness). Patients with a history of BCG vaccination and who were currently non-responsive to PPD as a result of illness could be rendered responsive to PPD by a subcutaneous injection of the amplifier fraction described in Example 2B, supra, using a 10- to 100-fold higher dose of the amplifier. Response to the antigen was not localized to the site of amplifier injection.

Systemic effects were dramatically demonstrated in a series of experiments using amplifier 6. A patient with a four-year history of sarcoidosis displayed extremely weak responses to all antigens. The following series of tests was performed. On day 1 of the test, the patient was administered tetanus toxoid, alone and in combination with amplifier 6 at several dilutions. The results, shown in Table 5, showed a weak erythematous response without induration to tetanus toxoid alone, and some slight amplification of response with amplifier 6. However, there was no induration at any of the test sites, indicating that the patient was not responding appropriately. On day 2 of the test, the patient received a subcutaneous injection of amplifier 6 from fraction 33 of the reverse-phase chromatography, in an amount equivalent to that extractable from approximately $2\times10^8$ leukocytes. On day 8 of the test, the patient was again challenged with tetanus toxoid, alone and in combination with several dilutions of amplifier 6, as before. However, this time there was a substantial response to amplifier 6, with some induration noted, as shown in Table 5. The results indicate a substantially increased responsiveness to the antigen, modulated by a systemic effect of the injection of amplifier 6 administered on day 2.

Further evidence of the systemic effect of amplifier 6 is provided by measurement of responsiveness of the patient's peripheral blood lymphocytes to activation in vitro. Lymphocyte activation is a well-known phenomenon. A sample of peripheral blood lymphocytes of normal individuals is induced to proliferate in cell culture by a variety of known activating agents, including various plant mitogens and phytohemagglutinin (hereinafter PHA). The rate of proliferation is manifested by uptake of $^3$H-thymidine from the culture medium into DNA of the dividing cells. The test procedure is described by Oppenheim, J. J., et al, in In Vitro Methods of Cell Mediated and Tumor Immunity (Bloom and David, Eds.), pp. 573—585, Academic Press, New York, N.Y. (1976).

Samples of peripheral lymphocytes were obtained from the patient on day 2 and day 8 of the above-described test, and assayed for response to a variety of activating agents. The results are shown in Table 6. Day 2 lymphocytes were well below the normal response level, while day 8 lymphocytes displayed normal or increased responsiveness to two of the three activators. Therefore, a substantial systemic response to the subcutaneous injection of amplifier 6 had occurred.

In the foregoing experiments, pokeweed mitogen was obtained from Gibco Laboratories, Grand Island, N.Y.; PHA and concanabalin A were obtained from Difco Laboratories, Detroit, Michigan. The results in Table 6 are expressed as counts per minute of $^3$H-thymidine uptake.

Example 3

On the basis of experimental results on individual volunteers, it is apparent that recipients having nongenetic anergic or hypoimmune conditions can be treated to increase immune responsiveness by the above-described amplifiers. Some of the experimental results on volunteers were obtained using material purified by Sephadex G-10 chromatography, presumably comprising a mixture of amplifiers. A preferred embodiment of the contemplated method of treatment is described as follows.

Amplifiers 1—6 are prepared and purified as described in Example 1, using reverse phase chromatography. Active fractions are pooled, lyophilized and redissolved in normal saline or other physiologically acceptable vehicle. An effective dose, e.g., 0.1 ml containing the equivalent amount of amplifiers 1—6 purified from $5\times10^7$ leukocytes, is injected subcutaneously. Increased immune responsiveness is monitored by the patient's reactivity to an antigen to which he is known to be sensitive, comparing reactivity before and after administering the amplifiers. The amplifiers are administered either individually, or in combination, depending upon the desired effects. The persistence of the systemic modulation produced by administration of the amplifiers varies from patient to patient, and must therefore be monitored periodically with a suitable sensitivity test, as described. Additional doses are administered as required to maintain a desired amplification of immunity based upon the professional judgement of the attending physician.

Example 4

The amplifiers, either singly or in combination, are used to produce an immune response to weak vaccines. Many pathogens, including several Staphylococcus varieties and fungi responsible for Histoplasmosis or Candidiasis, fail to provoke a strong immune response in certain patients. Moreover, there is no known satisfactory vaccine for conferring immunity on such patients. Such fungal infections are especially dangerous for patients subjected to cancer chemotherapy, or immunosuppressive drugs. By enhancing the patients' immune response to weak antigens, however, the concurrent administration of the described amplifiers, either singly or in combination, makes it possible to prepare vaccines against such pathogens. Patients about to receive chemotherapy, or transplant surgery, can thus be vaccinated prior to treatment to reduce their susceptibility to histoplasmosis or candidiasis.

**0 042 064**

The vaccine is preferably prepared by combining antigens of the desired pathogen, prepared according to known methods in the art to ensure adequate attenuation and sterility, with amplifiers 1—6, prepared as described in Example 1. The vaccine is administered by standard procedures.

Used as described, amplifiers 1—6 are expected to expand the scope of preventive measures in medicine, and to enlarge the range of potential immunity antigens.

Example 5

Severe skin reactions to poison ivy or other contact hypersensitivity reactions are preventable by treatment with S-suppressor. Such a treatment would proceed as follows, on the basis of the known fact that the major portion of contact dermatitis reaction, such as that of poison ivy or other allergens, is a DH reaction. S-suppressor would be purified as described in Example 1. The active fractions from the reverse phase chromatography would be pooled and lyophilized, then incorporated into a salve or ointment suitable for topical application, such as a cold-cream-base composition. Where the reaction is generalized, e.g., in tissue or organ transplantation, parenteral administration may be preferred. Since the yield of observed suppressor activity obtained from individual donors is variable, the applied dosage must be expressed in terms of activity units. One suppressor unit may be defined as the minimum amount necessary to reduce the diameter of induration in an individual's DH response, by 5 mm. Using this definition, the skin area to be treated should receive a dose at least one unit per 100 cm$^2$, with larger doses being administered if clinically indicated. Treatment is to be continued as long as there is danger of exposure, with reapplications at least every seven days, but more frequently if clinically indicated. The response of individual patients will vary depending upon their degree of sensitivity to the antigen, and their responsiveness to suppressor. Such compositions may also advantageously include steroids or other anti-inflammatory agents to enhance their therapeutic effect.

Example 6

Severe poison ivy and other contact skin reactions are preventable by the administration of L-suppressor, prepared as described in Example 1. Again, the known DH component of such skin reactions clearly indicates the effectiveness of such a therapeutic approach. Active fractions from hydroxylapatite chromatography should be pooled, lyophilized and incorporated into a salve or ointment composition suitable for topical application. Where the reaction is generalized e.g., in tissue or organ transplantation, parenteral administration may be preferred. Dosage and method of treatment follow essentially as described in Example 3.

The L-suppressor and S-suppressor may be applied separately, or mixed together in a single composition.

General concluding remarks

The above described modulators of the immune system are considered to the substances whose natural function is regulation of the immune response, directly with respect to cell mediated immunity and perhaps indirectly affecting humoral immunity as well. The substances have been prepared with a high degree of purity such that their properties have now been characterized and shown to be entirely and unexpectedly different from transfer factor and partial fractionations thereof reported in the prior art. The amplifiers and suppressors herein described are medically useful for the treatment of human patients suffering from a variety of hyperimmune and hypoimmune conditions. It is especially significant that these substances may be isolated from normal individuals, rather than from specific identified donors, so that large-scale purification from pooled sources is feasible. It will be understood that the modulators disclosed and claimed herein are defined in terms of their biological activities and physical properties and do not necessarily consist of single chemical substances.

# 0 042 064

### TABLE 1
Dilution studies of fractions 14 and 5
From reverse-phase chromatography

| Fraction #14 | 7 Hours | | 12 Hours | | 24 Hours | |
|---|---|---|---|---|---|---|
|  | Erythema | Induration | Erythema | Induration | Erythema | Induration |
| undil.+SK | 12×11 | 0 | 14×18 | 0 | 11×11 | 0 |
| $10^{-1}$+SK | 18×16 | 18×16 | 26×25 | 15×15 | 20×20 | 0 |
| $10^{-2}$+SK | 15×13 | 15×13 | 23×23 | 15×15 | 10×11 | 10×11 |
| $10^{-3}$+SK | 15×20 | 15×20 | 21×23 | 12×12 | 18×14 | 18×14 |
| $10^{-4}$+SK | 0 | 0 | 4×3 | 0 | 9×9 | 4×4 |
| SK Control | 0 | 0 | 15×15 | 6×6 | 9×11 | 9×11 |

| Fraction #5 | 7 Hours | | 12 Hours | | 24 Hours | |
|---|---|---|---|---|---|---|
|  | Erythema | Induration | Erythema | Induration | Erythema | Induration |
| Undil.+SK | 20×20 | 20×20 | 25×30 | 20×20 | 15×20 | 15×20 |
| $10^{-1}$+SK | 20×15 | 20×15 | 26×20 | 12×12 | 12×14 | 12×14 |
| $10^{-2}$+SK | 18×18 | 18×18 | 23×22 | 20×20 | 15×18 | 15×18 |
| $10^{-3}$+SK | 16×18 | 16×18 | 21×21 | 20×20 | 13×12 | 13×12 |
| $10^{-4}$+SK | 20×20 | 20×20 | 25×25 | 25×24 | 26×24 | 26×24 |
| SK Control | 0 | 0 | 2×3 | 0 | 7×6 | 4×4 |

SK=streptokinase

### TABLE 2
"L" Fraction from hydroxylapatite

| Fraction | 25 Hours Induration (mm) | 43 Hours Induration (mm) |
|---|---|---|
| 448 | 14×17 | 14×14 |
| 449 | 25×25 | 20×18 |
| 450 | 3×3 | 3×3 |
| 451 | 3×2 | 3×4 |
| 452 | 16×16 | 15×14 |
| 453 | 12×16 | 18×17 |
| 454 | 14×10 | 15×14 |
| Control PPD (2.5 units) | 26×24 | 16×13 |

TABLE 3

| Donor: TK Recipient: SS Fraction No. | 2 hr. | 4 hr. | mm Induration 8 hr. | 24 hr. | | 48 hr. |
|---|---|---|---|---|---|---|
| 33 | | | | 5 | Histo | 0 |
| 34 | | | | 0 | Histo | 0 |
| 33+PPD | | | | 25 | | 30 |
| 34+PPD | | | | 35 | | 50 |
| PPD | | | | 8 | | 30 |
| | | | | | Histo | 0 |

| Donor: TK Recipient: SJ Fraction No. | 2 hr. | 4 hr. | mm Induration 8 hr. | 24 hr. | | 48 hr. |
|---|---|---|---|---|---|---|
| 33 | 8 | 7 | 8 | 8 | Histo | 0 |
| 34 | 0 | 0 | 0 | 0 | Histo | 0 |
| 33+PPD | 7 | 7 | 20 | 20 | | 30 |
| 34+PPD | 0 | 5 | 3 | 35 | | 50 |
| PPD | 0 | 0 | 0 | 25 | | 20 |
| | | | | | Histo | 0 |

TABLE 4
A. Extent of dermal reaction at indicated time*

| Fraction | 5.5 hrs. | 12.5 hrs. | 24 hrs. |
|---|---|---|---|
| 32<br>32 ($10^{-2}$ dilution) | 96<br>120 | 36<br>360 | 15<br>173 |
| 35<br>35 ($10^{-2}$ dilution) | 90<br>132 | 156<br>428 | −14<br>129 |
| 37<br>37 ($10^{-2}$ dilution) | 36<br>132 | 75<br>356 | −7<br>159 |

B. Extent of dermal reaction at indicated time*

| Fraction | 4 hrs. | 11 hrs. |
|---|---|---|
| 33 ($10^{-1}$ dilution) | 8 | 41 |
| 33 ($10^{-2}$ dilution) | 41 | 48 |
| 33 ($10^{-3}$ dilution) | 99 | 255 |
| 33 ($10^{-4}$ dilution) | 89 | 167 |

* $(a \times b) - (a^* \times b^*)$ in mm²

### TABLE 5
#### A. Day 1 response
Lesion Dimensions in Millimeters

| | 10.5 hrs. | 23 hrs. | 45 hrs. |
|---|---|---|---|
| Tetanus Toxoid (TT) | 15×20 | 10×10 | 15×15 |
| TT+Amplifier 6 ($10^{-1}$ dilution) | 20×25 | 14×12 | 20×25 |
| TT+Amplifier 6 ($10^{-2}$ dilution) | 20×20 | 14×10 | 20×20 |
| TT+Amplifier 6 ($10^{-3}$ dilution) | 10×10 | 10×11 | 15×15 |
| TT+Amplifier 6 ($5×10^{-3}$ dilution) | 15×20 | 7×7 | 10×10 |

#### B. Day 8 response
Lesion dimensions in millimeters

| | 8 hrs. | 20 hrs. |
|---|---|---|
| TT (control) | 10×15 | 10×10 |
| TT+Amplifier 6 ($10^{-1}$ dilution) | { 40×36 } confluent | 10×18 |
| TT+Amplifier 6 ($10^{-2}$ dilution) | { 40×36 } confluent | 15×18 |
| TT+Amplifier 6 ($10^{-3}$ dilution) | 15×15 | 10×15 |
| TT+Amplifier 6 ($5×10^{-3}$ dilution) | 15×15 | 10×10 |

### TABLE 6
Lymphocyte activation
$^3$H-thymidine untake, cpm

| Activator | Patient/Day 2 | Normal | Patient/Day 8 |
|---|---|---|---|
| None (media control) | 419.0 | | 530.1 |
| Pokeweed Mitogen | 8,001.7 | >20,000 | 17,279.9 |
| Concanavalin A | 9,173.1 | >20,000 | 34,066.3 |
| PHA-P 1:50 | 20,208.3 | >40,000 | 27,899.2 |
| PHA-P 1:200 | 6,605.1 | | 7,257.1 |
| PHA-P 1:800 | 551.1 | | 1,710.3 |

**Claims for the Contracting States: BE, CH, DE, FR, GB, IT, LI, LU, NL, SE**

1. A "modulator of the immune system" derived from human leukocytes, purified substantially free of fluorescamine-reactive substances, having the activity of increasing or decreasing a delayed hypersensitivity skin response to antigens in a human being, characterized in that the modulator, termed "amplifier 1", having activity reaching a maximum with increasing modulator concentration, being dialyzable through a dialysis membrane having a nominal molecular weight cutoff of about 3,500 and said amplifier being reverse-phase chromatographically elutable from octadecyl silane ("elutable") by at least 15% (v/v) ethanol in water.

2. A composition for increasing responsiveness of the human immune system to said antigens, comprising amplifier 1, according to claim 1, in a pharmaceutically acceptable vehicle.

3. A modulator according to the preamble of claim 1, characterized in that it is termed "amplifier 2", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifier 1, by at least 45% (v/v) ethanol in water solution, and being essentially non-elutable by less than 45% (v/v) ethanol in water.

4. A composition for increasing responsiveness in a human immune system to said antigens comprising amplifier 2, according to claim 3, in a pharmaceutically acceptable vehicle.

5. A modulator according to the preamble of claim 1, characterized in that it is termed "amplifier 3", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifier 1 and amplifier 2, by at least 70% (v/v) ethanol in water solution, and being essentially non-elutable by less than 70% (v/v) ethanol in water.

6. A composition for increasing responsiveness in a human immune system to said antigens comprising amplifier 3, according to claim 5, in a pharmaceutically acceptable vehicle.

7. A modulator according to the preamble of claim 1, characterized in that it is termed "amplifier 4", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifiers 1, 2 and 3, by at least 80% (v/v) ethanol in water solution, and being essentially non-elutable by less than 80% (v/v) ethanol in water.

8. A composition for increasing responsiveness in a human immune system to said antigens comprising amplifier 4, according to claim 7, in a pharmaceutically acceptable vehicle.

9. A modulator according to the preamble of claim 1, characterized in that it is termed "amplifier 5", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifiers 1, 2, 3 and 4, by at least 89% (v/v) ethanol in water solution, and being essentially non-elutable by less than 89% (v/v) ethanol in water.

10. A composition for increasing responsiveness in a human immune system to said antigens comprising amplifier 5, according to claim 9, in a pharmaceutically acceptable vehicle.

11. A modulator according to the preamble of claim 1, characterized in that it is termed "amplifier 6", having activity that is maximal at an optimum concentration above which said activity is reduced, having a systemic modulating effect, being dialyzable through a dialysis membrane having a nominal molecular weight cutoff of about 3,500 and said amplifier being reverse-phase chromatographically elutable from octadecyl silane ("elutable") after eluation of amplifiers 1, 2, 3, 4 and 5, by 100% (v/v) ethanol, and being essentially non-elutable by ethanol diluted with water.

12. A composition for increasing responsiveness in a human immune system to said antigens comprising amplifier 6, according to claim 11, in a pharmaceutically acceptable vehicle.

13. A modulator according to the preamble of claim 1, characterized in that it is termed "L-suppressor", said suppressor being dialyzable through a dialysis membrane having a nominal molecular weight cutoff of about 12,000, but non-dialyzable through a dialysis membrane having a nominal molecular weight cutoff of about 3,500, said suppressor being reverse-phase chromatographically elutable from hydroxylapatite by 0.1 to 0.15 M aqueous ammonium bicarbonate.

14. A composition for decreasing responsiveness in a human immune system to said antigens comprising L-suppressor, according to claim 13, in a pharmaceutically acceptable vehicle suitable for topical or parenteral administration.

15. A modulator according to the preamble of claim 1, characterized in that it is termed "S-suppressor", said suppressor being dialysable through a dialysis membrane having a nominal molecular weight cutoff of about 3,500, and being reverse-phase chromatographically elutable from octadecyl silane ("elutable") by at least 67% (v/v) ethanol in water, essentially non-elutable in less than 67% (v/v) ethanol in water, and at least 90% elutable by at least 96% (v/v) ethanol in water.

16. A method of purifying from an extract of human leukocytes, amplifiers 1 to 6 and S-suppressor, and separating them from L-suppressor and other materials having amplifier, suppressor and inducer activities and from substantially all fluorescamine-reactive material, characterized by

1) dialyzing said extract through a dialysis membrane having a nominal molecular weight cutoff of about 3,500,
2) fractionating the dialysate by gel filtration, thereby producing a plurality of fractions,
3) pooling modulator-assayed fractions,
4) applying the pooled modulator-assayed fractions to a reverse-phase chromatography column, and
5) eluting the column with an ethanol-in-water gradient.

17. The method of claim 16, wherein said gradient includes ethanol in water from 15% (v/v) to 20% (v/v) and the material eluted in said range is collected.

18. The method of claim 16, wherein material eluting from the reverse-phase chromatography from 45% (v/v) ethanol in water to 53% (v/v) ethanol in water is collected.

19. The method of claim 16, wherein material from the reverse-phase chromatography eluting from between 70% (v/v) ethanol in water to 74% (v/v) ethanol in water is collected.

20. The method of claim 16, wherein material from reverse-phase chromatography eluting from 80% (v/v) ethanol in water to 84% (v/v) ethanol in water is collected.

21. The method of claim 16, wherein material from reverse-phase chromatography eluting from 89% (v/v) ethanol in water to 94% (v/v) ethanol in water is collected.

22. A method according to claim 16, comprising additionally the step of eluting the column with 100% ethanol, and the material eluted in said ethanol is collected.

23. A method according to claim 16, wherein material from reverse-phase chromatography eluting from between 96% (v/v) ethanol in water to 99% (v/v) ethanol in water is collected.

24. A method of purifying from an extract of human leukocytes, L-suppressor from amplifiers 1—6 and S-suppressor, and separating it from essentially all fluorescamine-reactive material, characterised by

1) dialyzing said extract through a first dialysis membrane having a nominal molecular weight cut-off of about 12,000,
2) dialyzing the first dialysate through a second dialysis membrane having a nominal molecular weight cutoff of about 3,500,
3) applying the retentate of the second dialysis to an hydroxylapatite chromatography column, and
4) collecting the fractions eluting from the column with ammonium bicarbonate gradient from 0.1M to 0.15M.

## Claims for the Contracting State: AT

1. A method for producing a "modulator of the immune system" derived from human leukocytes, purified substantially free of fluoroescamine-reactive substances, having the activity of increasing or decreasing a delayed hypersensitivity skin response to antigens in a human being, characterized in that the modulator, termed "amplifier 1", having activity reaching a maximum with increasing modulator concentration, being dialyzable through a dialysis membrane having a nominal molecular weight cutoff of about 3,500 and said amplifier being reverse-phase chromatographically elutable from octadecyl silane ("elutable") by at least 15% (v/v) ethanol in water.

2. A method for producing a composition for increasing responsiveness of the human immune system to said antigens, comprising amplifier 1, according to claim 1, in a pharmaceutically acceptable vehicle.

3. A method according to the preamble of claim 1, characterized in that it is termed "amplifier 2", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifier 1, by at least 45% (v/v) ethanol in water solution, and being essentially non-elutable by less than 45% (v/v) ethanol in water.

4. A method for producing a composition for increasing responsiveness in a human immune system to said antigens comprising amplifier 2, according to claim 3, in a pharmaceutically acceptable vehicle.

5. A method according to the preamble of claim 1, characterized in that it is termed "amplifier 3", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifier 1 and amplifier 2, by at least 70% (v/v) ethanol in water solution, and being essentially non-elutable by less than 70% (v/v) ethanol in water.

6. A method for producing a composition for increasing responsiveness in a human immune system to said antigens comprising amplifier 3, according to claim 5, in a pharmaceutically acceptable vehicle.

7. A method according to the preamble of claim 1, characterized in that it is termed "amplifier 4", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after eluation of amplifiers 1, 2 and 3, by at least 80% (v/v) ethanol in water solution, and being essentially non-elutable by less than 80% (v/v) ethanol in water.

8. A method for producing a composition for increasing responsiveness in a human immune system to said antigens comprising amplifier 4, according to claim 7, in a pharmaceutically acceptable vehicle.

9. A method according to the preamble of claim 1, characterized in that it is termed "amplifier 5", having activity that is maximal at an optimum concentration above which said activity is reduced, being dialyzable through a dialysis membrane having a nominal molecular weight cutoff of about 3,500 and said amplifier being reverse-phase chromatographically elutable from octadecyl silane ("elutable") after

16

# 0 042 064

eluation of amplifiers 1, 2, 3 and 4, by at least 89% (v/v) ethanol in water solution, and being essentially non-elutable by less than 89% (v/v) ethanol in water.

10. A method for producing a composition for increasing responsiveness in a human immune system to said antigens comprising amplifier 5, according to claim 9, in a pharmaceutically acceptable vehicle.

11. A method according to the preamble of claim 1, characterized in that it is termed "amplifier 6", having activity that is maximal at an optimum concentration above which said activity is reduced, having a systemic modulating effect, being dialyzable through a dialysis membrane having a nominal molecular weight cutoff of about 3,500 and said amplifier being reverse-phase chromatographically elutable from octadecyl silane ("elutable") after eluation of amplifiers 1, 2, 3, 4 and 5, by 100% (v/v) ethanol, and being essentially non-elutable by ethanol diluted with water.

12. A method for producing a composition for increasing responsiveness in a human immune system to said antigens comprising amplifier 6, according to claim 11, in a pharmaceutically acceptable vehicle.

13. A method according to the preamble of claim 1, characterized in that it is termed "L-suppressor", said suppressor being dialyzable through a dialysis membrane having a nominal molecular weight cutoff of about 12,000, but non-dialyzable through a dialysis membrane having a nominal molecular weight cutoff of about 3,500, said suppressor being reverse-phase chromatographically elutable from hydroxylapatite by 0.1 to 0.15 M aqueous ammonium bicarbonate.

14. A method for producing a composition for decreasing responsiveness in a human immune system to said antigens comprising L-suppressor, according to claim 13, in a pharmaceutically acceptable vehicle suitable for topical or parenteral administration.

15. A method according to the preamble of claim 1, characterized in that it is termed "S-suppressor", said suppressor being dialysable through a dialysis membrane having a nominal molecular weight cutoff of about 3,500, and being reverse-phase chromatographically elutable from octadecyl silane ("elutable") by at least 67% (v/v) ethanol in water, essentially non-elutable in less than 67% (v/v) ethanol in water, and at least 90% elutable by at least 96% (v/v) ethanol in water.

16. A method of purifying from an extract of human leukocytes, amplifiers 1 to 6 and S-suppressor, and separating them from L-suppressor and other materials having amplifier, suppressor and inducer activities and from substantially all fluorescamine-reactive material, characterized by

1) dialyzing said extract through a dialysis membrane having a nominal molecular weight cutoff of about 3,500,
2) fractionating the dialysate by gel filtration, thereby producing a plurality of fractions,
3) pooling modulator-assayed fractions,
4) applying the pooled modulator-assayed fractions to a reverse-phase chromatography column, and
5) eluting the column with an ethanol-in-water gradient.

17. The method of claim 16, wherein said gradient includes ethanol in water from 15% (v/v) to 20% (v/v) and the material eluted in said range is collected.

18. The method of claim 16, wherein material eluting from the reverse-phase chromatography from 45% (v/v) ethanol in water to 53% (v/v) ethanol in water is collected.

19. The method of claim 16, wherein material from the reverse-phase chromatography eluting from between 70% (v/v) ethanol in water to 74% (v/v) ethanol in water is collected.

20. The method of claim 16, wherein material from reverse-phase chromatography eluting from 80% (v/v) ethanol in water to 84% (v/v) ethanol in water is collected.

21. The method of claim 16, wherein material from reverse-phase chromatography eluting from 89% (v/v) ethanol in water to 94% (v/v) ethanol in water is collected.

22. A method according to claim 16, comprising additionally the step of eluting the column with 100% ethanol, and the material eluted in said ethanol is collected.

23. A method according to claim 16, wherein material from reverse-phase chromatography eluting from between 96% (v/v) ethanol in water to 99% (v/v) ethanol in water is collected.

24. A method of purifying from an extract of human leukocytes, L-suppressor from amplifiers 1—6 and S-suppressor, and separating it from essentially all fluorescamine-reactive material, characterized by

1) dialyzing said extract through a first dialysis membrane having a nominal molecular weight cutoff of about 12,000,
2) dialyzing the first dialysate through a second dialysis membrane having a nominal molecular weight cutoff of about 3,500,
3) applying the retentate of the second dialysis to an hydroxylapatite chromatography column, and
4) collecting the fractions eluting from the column with ammonium bicarbonate gradient from 0.1M to 0.15M.

**Patentansprüche fur die Vertragsstaaten: BE, CH, DE, FR, GB, IT, LI, LU, NL, SE**

1. "Modulator des Immunsystems", hergestellt aus menschlichen Leukozyten, gereinigt, im wesentlichen frei von Fluorescamin-aktiven Substanzen, die Aktivität zur Steigerung oder Verminderung

17

einer verzögerten Hypersensitivitäts-Haut-Reaktion auf Antigene in einem Menschen aufweisend, dadurch gekennzeichnet, daß der Modulator, "Amplifier 1" genannt, eine Aktivität aufweist, die mit wachsender Modulator-Konzentration ein Maximum erreicht, durch eine Dialyse-Membran mit einem nominellen Molekulargewichts-Cutoff von etwa 3500 dialysierbar ist und reversed-phase-chromatographisch von Octadecylsilan ("elutable") mit mindestens 15 Vol.-% Ethanol in Wasser eluierbar ist.

2. Zusammensetzung zur Steigerung der Empfindlichkeit des menschlichen Immunsystems auf Antigene, die Amplifier 1, entsprechend Anspruch 1, enthält.

3. Modulator nach dem Oberbegriff des Anspruchs 1, dadurch gekennzeichnet, daß er, "Amplifier 2" genannt, eine maximale Aktivität bei einer optimalen Konzentration, oberhalb welcher die Aktivität vermindert ist, aufweist, durch eine Dialyse-Membran mit einem nominellen Molekulargewichts-Cutoff von etwa 3500 dialysierbar ist und reversed-phase-chromatographisch, nach Elution des Amplifiers 1, von Octadecylsilan 45 Vol.-% Ethanol in Wasser eluierbar ist, jedoch nicht mit einer Lösung von weniger als 45 Vol.-% Ethanol in Wasser eluierbar ist.

4. Zusammensetzung zur Steigerung der Empfindlichkeit auf Antigene in einem menschlichen Immunsystem, die Amplifier 2, entsprechend Anspruch 3, in einem pharmazeutisch geeigneten Träger enthält.

5. Modulator entsprechend des Oberbegriff von Anspruch 1, dadurch gekennzeichnet, daß er, "Amplifier 3" genannt, eine maximale Aktivität bei einer optimalen Konzentration, oberhalb welcher die Aktivität vermindert ist, aufweist, durch eine Dialyse-Membran mit einem nominellen Molekulargewichts-Cutoff von etwa 3500 dialysierbar ist und der Amplifier 3 nach der Elution von Amplifier 1 und Amplifier 2 reversed-phase-chromatographisch von Octadecylsilan ("elutable") mit einer Lösung von mindestens 70 Vol.-% Ethanol in Wasser eluierbar ist und im wesentlichen bei einem Ehtanolgehalt von weniger als 70% in Wasser nicht eluierbar ist.

6. Zusammensetzung zur Steigerung der Empfindlichkeit in einem menschlichen Immunsystem auf Antigene, die Amplifier 3, nach Anspruch 5, in einem pharmazeutisch geeigneten Träger enthält.

7. Modulator entsprechend dem Oberbegriff von Anspruch 1, dadurch gekennzeichnet, daß er, "Amplifier 4" genannt, eine maximale Aktivität bei einer optimalen Konzentration, oberhalb der die Aktivität vermindert ist, aufweist, durch eine Dialyse-Membran mit einem nominellen Molekulargewichts-Cutoff von etwa ·3500 dialysierbar ist, und der Amplifier reversed-phase-chromatographisch von Octadecylsilan ("elutable") nach der Elution der Amplifier 1, 2 und 3, mit einer Lösung von mindestens 80 Vol.-% Ethanol in Wasser eluierbar ist und im wesentlichen mit weniger als 80 Vol.-% Ethanol in Wasser nicht eluierbar ist.

8. Zusammensetzung zur Steigerung der Empfindlichkeit in einem menschlichen Immunsystem auf Antigene, die Amplifier 4, entsprechend Anspruch 7, in einem pharmazeutisch geeigneten Träger enthält.

9. Modulator entsprechend dem Oberbegriff von Anspruch 1, dadurch gekennzeichnet, daß er, "Amplifier 5" genannt, eine maximale Aktivität bei einer optimalen Konzentration, oberhalb der die Aktivität vermindert ist, aufweist, dialysierbar ist durch eine Dialyse-Membran mit einem nominellen Molekulargewichts-Cutoff von etwa 3500 und der Amplifier 5 reversed-phase-chromatographisch von Octadecylsilan ("elutable") nach der Elution der Amplifier 1, 2, 3 und 4, mit einer Lösung von mindestens 89 Vol.-% Ethanol in Wasser eluierbar ist und im wesentlichen mit weniger als 89 Vol.-% Ethanol in Wasser nicht eluierbar ist.

10. Zusammensetzung zur Steigerung der Empfindlichkeit in einem menschlichen Immunsystem auf Antigene, die Amplifier 5, entspechend Anspruch 9, in einem pharmazeutisch geeigneten Träger enthält.

11. Modulator gemäß dem Oberbegriff des Anspruchs 1, dadurch gekennzeichnet, daß er, "Amplifier 6" genannt, eine maximale Aktivität bei einer optimalen Konzentration, oberhalb der die Aktivität vermindert ist, aufweist; einen Systemmodulierenden Effekt hat, durch eine Dialyse-Membran mit einem nominellen Molekulargewichts-Cutoff von etwa 3500 dialysierbar ist und nach der Elution der Amplifier 1, 2, 3, 4 und 5, reversed-phase-chromatographisch von Octadecylsilan ("elutable") mit 100 Vol.-% Ethanol eluierbar ist, jedoch im wesentlichen mit durch Wasser verdünntem Ethanol nicht eluierbar ist.

12. Zusammensetzung zur Steigerung der Empfindlichkeit auf Antigene in einem menschlichen Immunsystem, die Amplifier 6, entspechend em Anspruch 11, enthält.

13. Modulator gemäß dem Oberbegriff des Anspruchs 1, dadurch gekennzeichnet, daß er, "L-Suppressor" genannt, durch eine Dialyse-Membran mit einem nominellen Molekulargewichts-Cutoff von etwa 12 000 dialysierbar ist, jedoch nicht dialysierbar ist durch eine Dialyse-Membran mit einem nominellen Molekulargewichts-Cutoff von etwa 3500, und daß der Suppressor reversed-phase-chromatographisch von Hydroxylapatit mit 0,1 bis 0,15 molarer wässriger Ammoniumbikarbonatlösung eluierbar ist.

14. Zusammensetzung zur Verminderung der Empfindlichkeit auf Antigene in einem menschlichen Immunsystem, die L-Suppressor, entsprechend Anspruch 13, in einem pharmazeutisch geeigneten Träger enthält und zur örtlichen und parenteralen Verabreichung geeignet ist.

15. Modulator gemäß dem Oberbegriff des Anspruchs 1, dadurch gekennzeichnet, daß er, "S-Suppressor" genannt, durch eine Dialyse-Membran mit einem nominellen Molekulargewichts-Cutoff von etwa 3500 dialysierbar ist und reversed-phase-chromatographisch von Octadecylsilan ("elutable") mit einer Lösung von mindestens 67 Vol.-% Ethanol in Wasser eluierbar ist, jedoch im wesentlichen nicht eluierbar ist mit einer Lösung von weniger als 67 Vol.-% Ethanol in Wasser, und zu wenigstens 90% eluierbar mit wenigstens 96 Vol.-% Ethanol in Wasser.

16. Verfahren zur Reinigung der Amplifier 1 bis 6 und des S-Suppressors aus einem Extrakt von menschlichen Leukozyten und zu deren Abtennung vom L-Suppressor und anderen Materialien mit Amplifier-, Suppressor- und Inducer- Aktivitäten sowie von im wesentlichen allem Fluorescaminreaktiven Material, gekennzeichnet durch

1. Dialysieren des Extraktes durch eine Dialyse-Membran mit einem nominellen Molekulargewichts-Cutoff von etwa 3500;
2. Fraktionieren des Dialysates mittels Gel-Filtration, wodurch eine Vielzahl von Fraktionen entstehen;
3. Vereinigen Modulator-geprüfter Fraktionen;
4. Aufbringen der vereinigten Modulator-geprüften Fraktionen auf eine reversed-phase-chromatographie-Säule und
5. Eluieren der Säule mit einem Ethanol-in-Wasser-Gradienten.

17. Verfahren nach Anspruch 16, dadurch gekennzeichnet, daß der Gradient 15 Vol.-% bis 20 Vol.-% Ethanol in Wasser enthält und das in diesem Bereich eluierte Material gesammelt wird.

18. Verfahren nach Anspruch 16, dadurch gekennzeichnet, daß das Material, das bei der reversed-phase-Chromatographie mit von 45 Vol.-% Ethanol in Wasser bis 53 Vol.-% Ethanol in Wasser eluiert wird, gesammelt wird.

19. Verfahren nach Anspruch 16, dadurch gekennzeichnet, daß das Material, das bei der reversed-phase-Chromatographie von zwischen 70 Vol.-% Ethanol in Wasser und 74 Vol.-% Ethanol in Wasser eluiert wird, gesammelt wird.

20. Verfahren nach Anspruch 16, dadurch gekennzeichnet, daß das Material, das bei der reversed-phase-Chromatographie mit von 80 Vol.-% Ethanol in Wasser bis 84 Vol.-% Ethanol in Wasser eluiert wird, gesammelt wird.

21. Verfahren nach Anspruch 16, dadurch gekennzeichnet, daß das Material, das bei der reversed-phase-Chromatographie mit von 89 Vol.-% Ethanol in Wasser bis 94 Vol.-% Ethanol in Wasser eluiert wird, gesammelt wird.

22. Verfahren nach Anspruch 16, dadurch gekennzeichnet, daß es zusätzlich das Eluieren der Säule mit 100% Ethanol enthält und das mit Ethanol eluierte Material gesammelt wird.

23. Verfahren nach Anspruch 16, dadurch gekennzeichnet, daß das Material, das bei der reversed-phase-Chromatographie mit von 96 Vol.-% Ethanol in Wasser bis 99 Vol.-% Ethanol in Wasser eluiert wird, gesammelt wird.

24. Verfahren zur Reinigung des L-Suppressors aus einem Extrakt von menschlichen Leukozyten von Amplifiern 1 bis 6 und S-Suppressor und Abtrennung des L-Suppressors von im wesentlichen allem Fluorescamin-reaktiven Material, gekennzeichnet durch

1. Dialysieren des Extraktes durch eine erste Dialyse-Membran mit einem nominellen Molekulargewichts-Cutoff von etwa 12 000,
2. Dialysieren des ersten Dialysats durch eine zweite Dialysemembran mit einem nominellen Molekulargewichts-Cutoff von etwa 3500,
3. Aufbringen des Retentats der zweiten Dialyse auf eine Hydroxylapatit-Chromatographie-Säule und
4. Sammeln der Fraktionen, die von der Säule mit Ammoniumbikarbonat-Gradient von 0,1 m bis 0,15 m eluiert werden.

**Patentansprüche für den Vertragsstaat: AT**

1. Verfahren zur Herstellung eines "Modulators des Immunsystems", hergestellt aus menschlichen Leukozyten, gereinigt, im wesentlichen frei von Fluorescamin-aktiven Substanzen, die Aktivität zur Steigerung oder Verminderung einer verzögerten Hypersensitivitäts-Haut-Reaktion auf Antigene in einem Menschen aufweisend, dadurch gekennzeichnet, daß der Modulator, "Amplifier 1" genannt, eine Aktivität aufweist, die mit wachsender Modulator-Konzentration ein Maximum erreicht, durch eine Dialyse-Membran mit einem nominellen Molekulargewichts-Cutoff von etwa 3500 dialysierbar ist und reversed-phase-chromatographisch von Octadecylsilan ("elutable") mit mindestens 15 Vol.-% Ethanol in Wasser eluierbar ist.

2. Verfahren zur Herstellung einer Zusammensetzung zur Steigerung der Empfindlichkeit des menschlichen Immunsystems auf Antigene, die Amplifier 1, entspechend Anspruch 1, enthält.

3. Verfahren nach dem Oberbegriff des Anspruchs 1, dadurch gekennzeichnet, daß er, "Amplifier 2" genannt, eine maximale Aktivität bei einer optimalen Konzentration, oberhalb welcher die Aktivität vermindert ist, aufweist, durch eine Dialyse-Membran mit einem nominellen Molekulargewichts-Cutoff von etwa 3500 dialysierbar ist und reversed-phase-chromatographisch, nach Elution des Amplifiers 1, von Octadecylsilan 45 Vol.-% Ethanol in Wasser eluierbar ist, jedoch nicht mit einer Lösung von weniger als 45 Vol.-% Ethanol in Wasser eluierbar ist.

4. Verfahren zur Herstellung einer Zusammensetzung zur Steigerung der Empfindlichkeit auf Antigene in einem menschlichen Immunsystem, die Amplifier 2, entsprechend Anspruch 3, in einem pharmazeutisch geeigneten Träger enthält.

5. Verfahren entsprechend dem Oberbegriff von Anspruch 1, dadurch gekennzeichnet, daß er, "Amplifier 3" genannt, eine maximale Aktivität bei einer optimalen Konzentration, oberhalb welcher die Aktivität vermindert ist, aufweist, durch eine Dialyse-Membran mit einem nominellen Molekulargewichts-Cutoff von etwa 3500 dialysierbar ist und der Amplifier 3 nach der Elution von Amplifier 1 und Amplifier 2 reversed-phase-chromatographisch von Octadecylsilan ("elutable") mit einer Lösung von mindestens 70 Vol.-% Ethanol in Wasser eluierbar ist und im wesentlichen bei einem Ehtanolgehalt von weniger als 70% in Wasser nicht eluierbar ist.

6. Verfahren zur Herstellung einer Zusammensetzung zur Steigerung der Empfindlichkeit in einem menschlichen Immunsystem auf Antigene, die Amplifier 3, nach Anspruch 5, in einem pharmazeutisch geeigneten Träger enthält.

7. Verfahren entspechend dem Oberbegriff von Anspruch 1, dadurch gekennzeichnet, daß er, "Amplifier 4" genannt, eine maximale Aktivität bei einer optimalen Konzentration, oberhalb der die Aktivität vermindert ist, aufweist, durch eine Dialyse-Membran mit einem nominellen Molekulargewichts-Cutoff von etwa 3500 dialysierbar ist, und der Amplifier reversed-phase-chromatographisch von Octadecylsilan ("elutable") nach der Elution der Amplifier 1, 2 und 3, mit einer Lösung von mindestens 80 Vol.-% Ethanol in Wasser eluierbar ist und im wesentlichen mit weniger als 80 Vol.-% Ethanol in Wasser nicht eluierbar ist.

8. Verfahren zur Herstellung einer Zusammensetzung zur Steigerung der Empfindlichkeit in einem menschlichen Immunsystem auf Antigene, die Amplifier 4, entspechend Anspruch 7, in einem pharmazeutisch geeigneten Träger enthält.

9. Verfahren entsprechend dem Oberbegriff von Anspruch 1, dadurch gekennzeichnet, daß er, "Amplifier 5" genannt, eine maximale Aktivität bei einer optimalen Konzentration, oberhalb der die Aktivität vermindert ist, aufweist, dialysierbar ist durch eine Dialyse-Membran mit einem nominellen Molekulargewichts-Cutoff von etwa 3500 und der Amplifier 5 reversed-phase-chromatographisch von Octadecylsilan ("elutable") nach der Elution der Amplifier 1, 2, 3 und 4, mit einer Lösung von mindestens 89 Vol.-% Ethanol in Wasser eluierbar ist und im wesentlichen mit weniger als 89 Vol.-% Ethanol in Wasser nicht eluierbar ist.

10. Verfahren zur Herstellung einer Zusammensetzung zur Steigerung der Empfindlichkeit in einem menschlichen Immunsystem auf Antigene, die Amplifier 5, entspechend Anspruch 9, in einem pharmazeutisch geeigneten Träger enthält.

11. Verfahren gemäß dem Oberbegriff des Anspruchs 1, dadurch gekennzeichnet, daß er, "Amplifier 6" genannt, eine maximale Aktivität bei einer optimalen Konzentration, oberhalb der die Aktivität vermindert ist, aufweist; einen System-modulierenden Effekt hat, durch eine Dialyse-Membran mit einem nominellen Molekulargewichts-Cutoff von etwa 3500 dialysierbar ist und nach der Elution der Amplifier 1, 2, 3, 4 und 5, reversed-phase-chromatographisch von Octadecylsilan ("elutable") mit 100 Vol.-% Ethanol eluierbar ist, jedoch im wesentlichen mit durch Wasser verdünntem Ethanol nicht eluierbar ist.

12. Verfahren zur Herstellung einer Zusammensetzung zur Steigerung der Empfindlichkeit auf Antigene in einem menschlichen Immunsystem, die Amplifier 6, entsprechend em Anspruch 11, enthält.

13. Verfahren gemäß dem Oberbegriff des Anspruchs 1, dadurch gekennzeichnet, daß er, "L-Suppressor" genannt, durch eine Dialyse-Membran mit einem nominellen Molekulargewichts-Cutoff von etwa 12 000 dialysierbar ist, jedoch nicht dialysierbar ist durch eine Dialyse-Membran mit einem nominellen Molekulargewichts-Cutoff von etwa 3500, und daß der Suppressor reversed-phase-chromatographisch von Hydroxylapatit mit 0,1 bis 0,15 molarer wässriger Ammoniumbikarbonatlösung eluierbar ist.

14. Verfahren zur Herstellung einer Zusammensetzung zur Verminderung der Empfindlichkeit auf Antigene in einem menschlichen Immunsystem, die L-Suppressor, entsprechend Anspruch 13, in einem pharmazeutisch geeigneten Träger enthält und zur örtlichen und parenteralen Verabreichung geeignet ist.

15. Verfahren gemäß dem Oberbegriff des Anspruchs 1, dadurch gekennzeichnet, daß er, "S-Suppressor" genannt, durch eine Dialyse-Membran mit einem nominellen Molekulargewichts-Cutoff von etwa 3500 dialysierbar ist und reversed-phase-chromatographisch von Octadecylsilan ("elutable") mit einer Lösung von mindestens 67 Vol.-% Ethanol in Wasser eluierbar ist, jedoch im wesentlichen nicht eluierbar ist mit einer Lösung von weniger als 67 Vol.-% Ethanol in Wasser, und zu wenigstens 90% eluierbar mit wenigstens 96 Vol.-% Ethanol in Wasser.

16. Verfahren zur Reinigung der Amplifier 1 bis 6 und des S-Suppressors aus einem Extrakt von menschlichen Leukozyten und zu deren Abtennung von L-Suppressor und anderen Materialien mit Amplifier-, Suppressor- und Inducer-Aktivitäten sowie von im wesentlichen allem Fluorescamin-reaktiven Material, gekennzeichnet durch

1. Dialysieren des Extraktes durch eine Dialyse-Membran mit einem nominellen Molekulargewichts-Cutoff von etwa 3500;
2. Fraktionieren des Dialysates mittels Gel-Filtration, wodurch eine Vielzahl von Fraktionen entstehen;
3. Vereinigen Modulator-geprüfter Fraktionen;

# 0 042 064

4. Aufbringen der vereinigten Modulator-geprüften Fraktionen auf eine reversed-phase-chromatographie-Säule und

5. Eluieren der Säule mit einem Ethanol-in-Wasser-Gradienten.

17. Verfahren nach Anspruch 16, dadurch gekennzeichnet, daß der Gradient 15 Vol.-% bis 20 Vol.-% Ethanol in Wasser enthält und das in diesem Bereich eluierte Material gesammelt wird.

18. Verfahren nach Anspruch 16, dadurch gekennzeichnet, daß das Material, das bei der reversed-phase-Chromatographie mit von 45 Vol.-% Ethanol in Wasser bis 53 Vol.-% Ethanol in Wasser eluiert wird, gesammelt wird.

19. Verfahren nach Anspruch 16, dadurch gekennzeichnet, daß das Material, das bei der reversed-phase-Chromatographie von zwischen 70 Vol.-% Ethanol in Wasser und 74 Vol.-% Ethanol in Wasser eluiert wird, gesammelt wird.

20. Verfahren nach Anspruch 16, dadurch gekennzeichnet, daß das Material, das bei der reversed-phase-Chromatographie mit von 80 Vol.-% Ethanol in Wasser bis 84 Vol.-% Ethanol in Wasser eluiert wird, gesammelt wird.

21. Verfahren nach Anspruch 16, dadurch gekennzeichnet, daß das Material, das bei der reversed-phase-Chromatographie mit von 89 Vol.-% Ethanol in Wasser bis 94 Vol.-% Ethanol in Wasser eluiert wird, gesammelt wird.

22. Verfahren nach Anspruch 16, dadurch gekennzeichnet, daß es zusätzlich das Eluieren der Säule mit 100% Ethanol enthält und das mit Ethanol eluierte Material gesammelt wird.

23. Verfahren nach Anspruch 16, dadurch gekennzeichnet, daß das Material, das bei der reversed-phase-Chromatographie mit von 96 Vol.-% Ethanol in Wasser bis 99 Vol.-% Ethanol in Wasser eluiert wird, gesammelt wird.

24. Verfahren zur Reinigung des L-Suppressors aus einem Extrakt von menschlichen Leukozyten von Amplifiern 1 bis 6 und S-Suppressor und Abtrennung des L-Suppressors von im wesentlichen allem Fluorescamin-reaktiven Material, gekennzeichnet durch

1. Dialysieren des Extraktes durch eine erste Dialyse-Membran mit einem nominellen Molekulargewichts-Cutoff von etwa 12 000,

2. Dialysieren des ersten Dialysats durch eine zweite Dialysemembran mit einem nominellen Molekulargewichts-Cutoff von etwa 3500,

3. Aufbringen des Retentats der zweiten Dialyse auf eine Hydroxylapatit-Chromatographie-Säule und

4. Sammeln der Fraktionen, die von der Säule mit Ammoniumbikarbonat-Gradient von 0,1 m bis 0,15 m eluiert werden.

**Revendications pour les Etats Contractants: BE, CH, DE, FR, GB, IT, LI, LU, NL, SE**

1. "Modulateur du système immunitaire" dérivé de leucocytes humains, purifié et pratiquement dépourvu de substances réagissant à la fluorescamine, ayant l'activité d'augmenter ou de diminuer une réponse dermique d'hypersensibilité différée à des antigènes chez un être humain, caractérisé en ce qu'il est appelé "amplificateur 1", ayant une activité atteignant un maximum lorsque la concentration de modulateurs augmente, pouvant être dialysé à travers une membrane de dialyse ayant une coupure nominale de poids moléculaire d'environ 3500 et pouvant être élué par chromatographie en phase inverse de l'octadécyl silane par une solution aqueuse contenant au moins 15% (volume/volume) d'éthanol.

2. Composition pour augmenter la réponse du système immunitaire humain à ces antigènes, contenant l'amplificateur 1, selon la revendication 1, dans un véhicule pharmaceutiquement acceptable.

3. Modulateur selon le préambule de la revendication 1, caractérisé en ce qu'il est appelé "amplificateur 2", ayant une activité qui atteint un maximum pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution de l'amplificateur 1, par une solution aqueuse contenant au moins 45% (v/v) d'éthanol et étant essentiellement non éluable par une solution aqueuse contenant moins de 45% (v/v) d'éthanol.

4. Composition pour augmenter la réponse d'un système immunitaire humain à ces antigènes, contenant l'amplificateur 2, selon la revendication 3, dans un véhicule pharmaceutiquement acceptable.

5. Modulateur selon le préambule de la revendication 1, caractérisé en ce qu'il est appelé "amplificateur 3", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution de l'amplificateur 1 et de l'amplificateur 2, par une solution aqueuse contenant au moins 70% (v/v) d'éthanol, et étant essentiellement non éluable par une solution aqueuse contenant moins de 70% (v/v) d'éthanol.

6. Composition pour augmenter la réponse du système immunitaire humain à ces antigènes, contenant l'amplificateur 3, selon la revendication 5, dans un véhicule pharmaceutiquement acceptable.

7. Modulateur selon la préambule de la revendication 1, caractérisé en ce qu'il est appelé

21

"amplificateur 4", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution des amplificateurs 1, 2 et 3, et par une solution aqueuse contenant au moins 80% (v/v) d'éthanol et étant essentiellement non éluable par une solution aqueuse contenant moins de 80% (v/v) d'éthanol.

8. Composition pour augmenter la réponse du système immunitaire humain à ces antigènes contenant l'amplificateur 4, selon la revendication 7, dans un véhicule pharmaceutiquement acceptable.

9. Modulateur selon le préambule de la revendication 1, caractérisé en ce qu'il est appelé "amplificateur 5", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution des amplificateurs 1, 2, 3 et 4, par une solution aqueuse contenant au moins 89% (v/v) d'éthanol, et étant essentiellement non éluable par une solution aqueuse contenant moins de 89% (v/v) d'éthanol.

10. Composition pour augmenter la réponse du système immunitaire humain à ces antigènes, contenant l'amplificateur 5, selon la revendication 9, dans un véhicule pharmaceutiquement acceptable.

11. Modulateur selon le préambule de la revendication 1, caractérisé en ce qu'il est appelé "amplificateur 6", ayant une activité qui est maximale pour une concentration optimale au-dessus de laquelle cette activité est réduite, ayant un effet modulateur systémique, pouvant être dialysé à travers une membrane de dialyse ayant une coupure nominale de poids moléculaire d'environ 3500, et pouvant être élué par chromatographie en phase inverse de l'octadécyl silane après élution des amplificateurs 1, 2, 3, 4 et 5 par 100% d'éthanol, et étant essentiellement non éluable par une solution aqueuse d'éthanol.

12. Composition pour augmenter la réponse du système immunitaire humain à ces antigènes, contenant l'amplificateur 6, selon la revendication 11, dans un véhicule pharmaceutiquement acceptable.

13. Modulateur selon le préambule de la revendication 1, caractérisé en ce qu'il est appelé "suppresseur L", ce suppresseur pouvant être dialysé à travers une membrane de dialyse ayant une coupure nominale de poids moléculaire d'environ 12000, mais ne pouvant pas être dialysé à travers une membrane de dialyse ayant une coupure nominale de poids moléculaire d'environ 3500, ce suppresseur pouvant être élué par chromatographie en phase inverse de l'apatite hydroxylée par 0,1 à 0,15 M de bicarbonate d'ammonium aqueux.

14. Composition pour diminuer la réponse du système immunitaire humain à ces antigènes, contenant le suppresseur L selon la revendication 13, dans un véhicule pharmaceutiquement acceptable, approprié pour une administration topique ou par voie parentérale.

15. Modulateur selon le préambule de la revendication 1, caractérisé en ce qu'il est appelé "suppresseur S", ce suppresseur pouvant être dialysé à travers une membrane de dialyse ayant une coupure nominale de poids moléculaire d'environ 3500 et pouvant être élué par chromatographie en phase inverse de l'octadécyl silane par une solution aqueuse contenant au moins 67% (v/v) d'éthanol, étant essentiellement non éluable dans une solution aqueuse contenant moins de 67% (v/v) d'éthanol et pouvant être élué à au moins 90% par une solution aqueuse contenant au moins 96% (v/v) d'éthanol.

16. Procédé pour purifier à partir d'un extrait de leucocytes humains des amplificateurs 1 à 6 et le suppresseur S et pour les séparer du suppresseur L et d'autres substances ayant des activités d'amplificateur, de suppresseur et d'inducteur, et à partir de pratiquement toutes les substances pouvant réagir à la fluorescamine, caractérisé en ce que:

1) on dialyse cet extrait à travers une membrane de dialyse ayant une coupure nominale de poids moléculaire d'environ 3500,
2) on fractionne le dialysat par filtration sur gel, produisant ainsi une multiplicité de fractions,
3) on groupe les fractions essayées en tant que modulateurs,
4) on applique les fractions essayées en tant que modulateurs groupées sur une colonne de chromatographie en phase inverse, et
5) on élue la colonne avec un gradient d'éthanol dans l'eau.

17. Procédé selon la revendication 16, dans lequel ce gradient comprend de 15% (v/v) à 20% (v/v) d'éthanol dans l'eau et la substance éluée dans cette plage est recueillie.

18. Procédé selon la revendication 16, dans lequel la substance éluant de la chromatographie en phase inverse de 45% (v/v) d'éthanol dans l'eau à 53% (v/v) d'éthanol dans l'eau est recueillie.

19. Procédé selon la revendication 16, dans lequel la substance provenant de la chromatographie en phase inverse éluant d'entre 70% (v/v) d'éthanol dans l'eau à 74% (v/v) d'éthanol dans l'eau est recueillie.

20. Procédé selon la revendication 16, dans lequel la substance provenant de la chromatographie en phase inverse éluant de 80% (v/v) d'éthanol dans l'eau à 84% (v/v) d'éthanol dans l'eau est recueillie.

21. Procédé selon la revendication 16, dans lequel la substance provenant de la chromatographie en phase inverse et éluant de 89% (v/v) d'éthanol dans l'eau à 94% (v/v) d'éthanol dans l'eau est recueillie.

22. Procédé selon la revendication 16, comprenant en outre le stade dans lequel on élue la colonne avec 100% d'éthanol et on recueille de produit élué dans l'éthanol.

22

**0 042 064**

23. Procédé selon la revendication 16, dans lequel la substance en provenance de la chromatographie en phase inverse éluant d'entre 96% (v/v) d'éthanol dans l'eau à 99% (v/v) d'éthanol dans l'eau est recueillie.

24. Procédé pour purifier à partir d'un extrait de leucocytes humains le suppresseur L des amplificateurs 1 à 6 et du suppresseur S, et pour le séparer d'essentiellement toutes les substances réagissant à la fluorescamine, caractérisé en ce que:

1) on dialyse cet extrait à travers une première membrane de dialyse ayant une coupure nominale de poids moléculaire d'environ 12000;

2) on dialyse le premier dialysat à travers une deuxième membrane de dialyse ayant une coupure nominale de poids moléculaire d'environ 3500;

3) on applique la quantité retenue du deuxième dialysat à une colonne de chromatographie sur apatite hydroxylée, et

4) on recueille une fraction éluant de la colonne avec un gradient de bicarbonate d'ammonium compris entre 0,1 M et 0,15 M.

**Revendications pour l'Etat Contractant AUTRICHE**

1. Procédé pour la production d'un "modulateur du système immunitaire" dérivé de le leucocytes humains, purifié et pratiquement dépourvu de substances réagissant à la fluorescamine, ayant l'activité d'augmenter ou de diminuer une réponse dermique d'hypersensibilité différée à des antigènes chez un être humain, caractérisé en ce qu'il est appelé "amplificateur 1", ayant une activité atteignant un maximum lorsque la concentration de modulateurs augmente, pouvant être dialysé à travers une membrane de dialyse ayant une coupure nominale de poids moléculaire d'environ 3500 et pouvant être élué par chromatographie en phase inverse de l'octadécyl silane par une solution aqueuse contenant au moins 15% (volume/volume) d'éthanol.

2. Procédé pour la production d'une composition pour augmenter la réponse du système immunitaire humain à ces antigènes, contenant l'amplificateur 1, selon la revendication 1, dans un véhicule pharmaceutiquement acceptable.

3. Procédé selon le préambule de la revendication 1, caractérisé en ce qu'il est appelé "amplificateur 2", ayant une activité qui atteint un maximum pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution de l'amplificateur 1, par une solution aqueuse contenant au moins 45% (v/v) d'éthanol et étant essentiellement non éluable par une solution aqueuse contenant moins de 45% (v/v) d'éthanol.

4. Procédé pour la production d'une composition pour augmenter la réponse d'un système immunitaire humain à ces antigènes, contenant l'amplificateur 2, selon la revendication 3, dans un véhicule pharmaceutiquement acceptable.

5. Procédé selon le préambule de la revendication 1, caractérisé en ce qu'il est appelé "amplificateur 3", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution de l'amplificateur 1 et de l'amplificateur 2, par une solution aqueuse contenant au moins 70% (v/v) d'éthanol, et étant essentiellement non éluable par une solution aqueuse contenant moins de 70% (v/v) d'éthanol.

6. Procédé pour la production d'une composition pur augmenter la réponse du système immunitaire humain à ces antigènes, contenant l'amplificateur 3, selon la revendication 5, dans un véhicule pharmaceutiquement acceptable.

7. Procédé selon le préambule de la revendication 1, caractérisé en ce qu'il est apprelé "amplificateur 4", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution des amplificateurs 1, 2 et 3, et par une solution aqueuse contenant au moins 80% (v/v) d'éthanol et étant essentiellement non éluable par une solution aqueuse contenant moins de 80% (v/v) d'éthanol.

8. Procédé pour la production d'une composition pour augmenter la réponse du système immunitaire humain à ces antigènes, contenant l'amplificateur 4, selon la revendication 7, dans un véhicule pharmaceutiquement acceptable.

9. Procédé selon le préambule de la revendication 1, caractérisé en ce qu'il est appelé "amplificateur 5", ayant une activité qui est maximale pour une concentration optimale au-dessus de laquelle cette activité est réduite, pouvant être dialysé à travers une membrane de dialyse ayant une coupure nominale de poids moléculaire d'environ 3500 et pouvant être élué par chromatographie en phase inverse de l'octadécyl silane après élution des amplificateurs 1, 2, 3 et 4, par une solution aqueuse contenant au moins

23

# 0 042 064

89% (v/v) d'éthanol, et étant essentiellement non éluable par une solution aqueuse contenant moins de 89% (v/v) d'éthanol.

10. Procédé pour la production d'une composition pour augmenter la réponse du système immunitaire humain à ces antigènes, contenant l'amplificateur 5, selon la revendication 9, dans un véhicule pharmaceutiquement acceptable.

11. Procédé selon le préambule de la revendication 1, caractérisé en ce qu'il est appelé "amplificateur 6", ayant une activité qui est maximale pour une concentration optimale au-dessus de laquelle cette activité est réduite, ayant un effet modulateur systémique, pouvant être dialysé à travers une membrane de dialyse ayant une coupure nominale de poids moléculaire d'environ 3500, et pouvant être élué par chromatographie en phase inverse de l'octadécyl silane après élution des amplificateurs 1, 2, 3, 4 et 5 par 100% d'éthanol, et étant essentiellement non éluable par une solution aqueuse d'éthanol.

12. Procédé pour la production d'une composition pour augmenter la réponse du système immunitaire humain à ces antigènes, contenant l'amplificateur 6, selon la revendication 11, dans un véhicule pharmaceutiquement acceptable.

13. Procédé selon le préambule de la revendication 1, caractérisé en ce qu'il est appelé "suppresseur L", ce suppresseur pouvant être dialysé à travers une membrane de dialyse ayant une coupure nominale de poids moléculaire d'environ 12000, mais ne pouvant pas être dialysé à travers une membrane de dialyse ayant une coupure nominale de poids moléculaire d'environ 3500, ce suppresseur pouvant être élué par chromatographie en phase inverse de l'apatite hydroxylée par 0,1 à 0,15 M de bicarbonate d'ammonium aqueux.

14. Procédé pour la production d'une composition pour diminuer la réponse du système immunitaire humain à ces antigènes, contenant le suppresseur L selon la revendication 13, dans un véhicule pharmaceutiquement acceptable, approprié pour une administration topique ou par voie parentérale.

15. Procédé selon le préambule de la revendication 1, caractérisé en ce qu'il est appelé "suppresseur S", ce suppresseur pouvant être dialysé à travers une membrane de dialyse ayant une coupure nominale de poids moléculaire d'environ 3500 et pouvant être élué par chromatographie en phase inverse de l'octadécyl silane par une solution aqueuse contenant au moins 67% (v/v) d'éthanol, étant essentiellement non éluable dans une solution aqueuse contenant moins de 67% (v/v) d'éthanol et pouvant être élué à au moins 90% par une solution aqueuse contenant au moins 96% (v/v) d'éthanol.

16. Procédé pour purifier à partir d'un extrait de leucocytes humains des amplificateurs 1 à 6 et le suppresseur S et pour les séparer du suppresseur L et d'autres substances ayant des activités d'amplificateur, de suppresseur et d'inducteur, et à partir de pratiquement toutes les substances pouvant réagir à la fluorescamine, caractérisé en ce que:

1) on dialyse cet extrait à travers une membrane de dialyse ayant une coupure nominale de poids moléculaire d'environ 3500,
2) on fractionne le dialysat par filtration sur gel, produisant ainsi une multiplicité de fractions,
3) on groupe les fractions essayées en tant que modulateurs,
4) on applique les fractions essayées en tant que modulateurs groupées sur une colonne de chromatographie en phase inverse, et
5) on élue la colonne avec un gradient d'éthanol dans l'eau.

17. Procédé selon la revendication 16, dans lequel ce gradient comprend de 15% (v/v) à 20% (v/v) d'éthanol dans l'eau et la substance éluée dans cette plage est recueillie.

18. Procédé selon la revendication 16, dans lequel la substance éluant de la chromatographie en phase inverse de 45% (v/v) d'éthanol dans l'eau à 53% (v/v) d'éthanol dans l'eau est recueillie.

19. Procédé selon la revendication 16, dans lequel le substance provenant de la chromatographie en phase inverse éluant d'entre 70% (v/v) d'éthanol dans l'eau à 74% (v/v) d'éthanol dans l'eau est recueillie.

20. Procédé selon la revendication 16, dans lequel la substance provenant de la chromatographie en phase inverse éluant de 80% (v/v) d'éthanol dans l'eau à 84% (v/v) d'éthanol dans l'eau est recueillie.

21. Procédé selon la revendication 16, dans lequel la substance provenant de la chromatographie en phase inverse et éluant de 89% (v/v) d'éthanol dans l'eau à 94% (v/v) d'éthanol dans l'eau est recueillie.

22. Procédé selon la revendication 16, comprenant en outre le stade dans lequel on élue la colonne avec 100% d'éthanol et on recueillie le produit élué dans l'éthanol.

23. Procédé selon la revendication 16, dans lequel la substance en provenance de la chromatographie en phase inverse éluant d'entre 96% (v/v) d'éthanol dans l'eau à 99% (v/v) d'éthanol dans l'eau est recueillie.

24. Procédé pour purifier à partir d'un extrait de leucocytes humains le suppresseur L des amplificateurs 1 à 6 et du suppresseur S, et pour le séparer d'essentiellement toutes les substances réagissant à la fluorescamine, caractérisé en en ce que:

1) on dialyse cet extrait à travers une première membrane de dialyse ayant une coupure nominale de poids moléculaire d'environ 12000;
2) on dialyse le premier dialysat à travers une deuxième membrane de dialyse ayant une coupure nominale de poids moléculaire d'environ 3500;

24

**0 042 064**

3) on applique la quantité retenue du deuxième dialysat à une colonne de chromatographie sur apatite hydroxylée, et

4) on recueille une fraction éluant de la colonne avec un gradient de bicarbonate d'ammonium compris entre 0,1 M et 0,15 M.

FIG. I

0 042 064

FIG. 2

FIG.3

24 HRS. AFTER INJECTION
$10^{-1}$ DILUTION

FIG. 4

0 042 064